(19) Europäisches Patentamt / European Patent Office / Office européen des brevets

(11) **EP 4 245 322 A1**

(12) **EUROPEAN PATENT APPLICATION**
published in accordance with Art. 153(4) EPC


(43) Date of publication:
**20.09.2023 Bulletin 2023/38**

(21) Application number: **21891940.5**

(22) Date of filing: **11.11.2021**

(51) International Patent Classification (IPC):
***A61K 47/68*** (2017.01) ***A61K 31/4745*** (2006.01)
***A61K 39/395*** (2006.01) ***A61P 35/00*** (2006.01)

(52) Cooperative Patent Classification (CPC):
**A61K 31/4745; A61K 39/395; A61K 47/68; A61P 35/00**

(86) International application number:
**PCT/JP2021/041496**

(87) International publication number:
**WO 2022/102695 (19.05.2022 Gazette 2022/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.11.2020 JP 2020188910**



(71) Applicant: **Daiichi Sankyo Company, Limited**
**Tokyo 103-8426 (JP)**

(72) Inventor: **YAMATO Michiko**
**Tokyo 103-8426 (JP)**

(74) Representative: **Marks & Clerk LLP**
**15 Fetter Lane**
**London EC4A 1BW (GB)**


Remarks:
The complete document including Reference Table(s) and the Sequence Listing(s) can be downloaded from the EPO website

(54) **TREATMENT FOR MESOTHELIOMA THROUGH ADMINISTRATION OF ANTI-B7-H3 ANTIBODY-DRUG CONJUGATE**


(57) A therapeutic agent for mesothelioma comprising, as an active component, an anti-B7-H3 antibody-drug conjugate in which a drug-linker represented by the following formula,
wherein A represents a connecting position to an anti-B7-H3 antibody; is conjugated to the anti-B7-H3 antibody via a thioether bond, and/or a method of treatment for mesothelioma, comprising administering the anti-B7-H3 antibody-drug conjugate to a subject in need of treatment for mesothelioma.

[Formula 1]

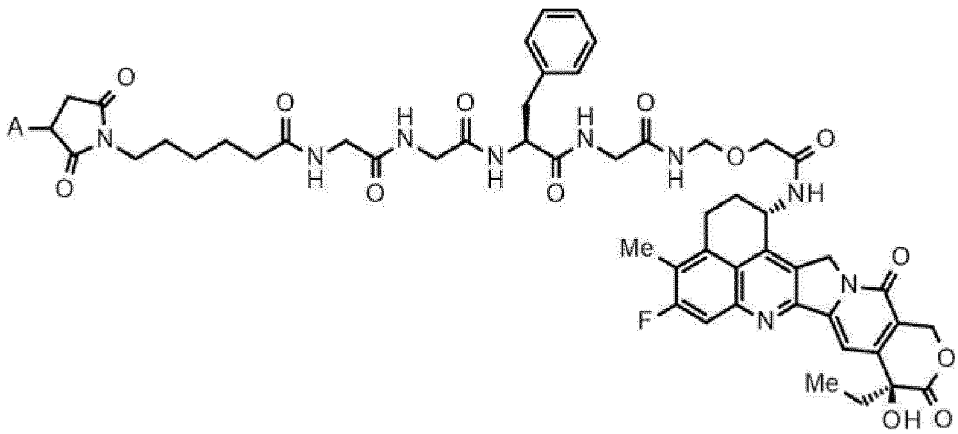


EP 4 245 322 A1

## Description

### Technical Field

The present invention relates to a therapeutic agent for mesothelioma comprising an anti-B7-H3 antibody-drug conjugate, and/or a method of treatment for mesothelioma comprising administering an anti-B7-H3 antibody-drug conjugate to a subject.

### Background Art

Mesothelioma is a malignant tumor that develops from mesothelial cells. Known origin sites are pleura, peritoneum, pericardium, tunica vaginalis testis, and the like, and mesotheliomas are classified into diffuse malignant mesothelioma, solitary malignant mesothelioma, and well-differentiated papillary mesothelioma (Non Patent Reference 1). Histologically, mesotheliomas are classified into the three types of epithelioid, sarcomatoid, and biphasic (Non Patent Reference 1).

Asbestos is known as one of the causes of developing mesothelioma. Mesothelioma distinctively has a long incubation period with onset over an incubation period of 30 years to 50 years since exposure. In the 1970s and 1980s, during which the use of asbestos was banned, or strictly regulated, mesothelioma incidence rates decreased in Australia, America, and western European countries, but the total death toll did not decrease (Non Patent Reference 2).

Methods of treatment for malignant pleural mesothelioma include (1) operative therapy (surgical therapy), (2) chemotherapy (anticancer agent therapy), and/or (3) radiotherapy. However, most patients are ineligible for surgical therapy on the grounds of extent of a disease, age, comorbidities, or poor general condition, and chemotherapy is alternatively used as a symptomatic therapy. The combination therapy of cisplatin and pemetrexed approved in 2004 by the U.S. Food and Drug Administration (FDA) is the standard therapy for mesothelioma. There is a report of increased efficacy by the addition of bevacizumab compared to the single use of the chemotherapy, but this therapy has not been approved by the FDA. Recently, several clinical studies using immune checkpoint inhibitors have been conducted, and effects were reported to be observed in 20% to 25% of mesothelioma patients (Non Patent Reference 2). However, approval has not yet been obtained.

B7-H3, one of the B7 family members expressed in antigen-presenting cells as a co-stimulator, is considered to act on receptors on T cells and enhance or suppress immune effects (Non Patent Reference 3).

B7-H3 is a single transmembrane protein and has two variants. B7-H3 variant 1 (4Ig-B7-H3) contains two each of V- or C-like Ig domains, and B7-H3 variant 2 (2Ig-B7-H3) contains one each of V- or C-like Ig domain (Non Patent Reference 4).

An antibody-drug conjugate (ADC) having a drug with cytotoxicity conjugated to an antibody capable of binding to an antigen expressed on the surface of cancer cells and cellular internalization, can deliver the drug selectively to cancer cells and can thus be expected to cause accumulation of the drug within cancer cells and to kill the cancer cells (Non-Patent References 5 to 9).

As one such antibody-drug conjugate, an antibody-drug conjugate comprising an anti-B7-H3 antibody and a derivative of exatecan, which is a topoisomerase I inhibitor, as its components is known (Patent References 1,2) .

[Citation List]

[Patent Literature]

[Patent Reference 1] International Publication No. WO 2014/057687
[Patent Reference 2] International Publication No. WO 2017/002776

[Non Patent Literature]

[Non Patent Reference 1] Timothy A Yap., et al., Nat Rev Cancer. 2017; 17(8): 475-488.
[Non Patent Reference 2] Carbone M., et al., Ca Cancer J Clin 2019; 69: 402-429.
[Non Patent Reference 3] Picarda E., et al., Clin Cancer Res. 2016; 22(14): 3425-31.
[Non Patent Reference 4] Ling V., et al., Genomics. 2003; 82(3): 365-77.
[Non Patent Reference 5] Ducry L., et al., Bioconjugate Chem. (2010) 21, 5-13.

[Non Patent Reference 6] Alley S. C., et al., Current Opinion in Chemical Biology (2010) 14, 529-537.
[Non Patent Reference 7] Damle N. K., Expert Opin. Biol. Ther. (2004) 4, 1445-1452.
[Non Patent Reference 8] Senter P. D., et al., Nature Biotechnology (2012) 30, 631-637.
[Non Patent Reference 9] Howard A., et al., J Clin Oncol 29: 398-405.

[Summary of Invention]

[Technical Problem]

The present invention provides a therapeutic agent and a method of treatment for mesothelioma.

Solution to Problem

The present inventors have found that an anti-B7-H3 antibody-drug conjugate exhibits an excellent antitumor effect on mesothelioma.

Thus, the present invention provides the following [1] to [88] .

[1] A therapeutic agent for mesothelioma comprising, as an active component, an anti-B7-H3 antibody-drug conjugate in which a drug-linker represented by the following formula:

[Formula 1]

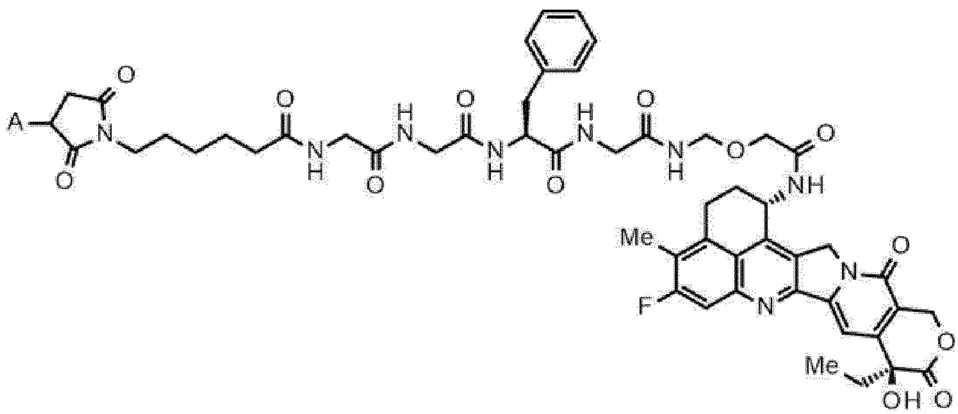

wherein A represents a connecting position to an anti-B7-H3 antibody;
is conjugated to the anti-B7-H3 antibody via a thioether bond.

[2] The therapeutic agent according to [1], wherein the mesothelioma is at least one selected from the group consisting of pleural mesothelioma, peritoneal mesothelioma, pericardial mesothelioma, and tunica vaginalis testis mesothelioma.

[3] The therapeutic agent according to [1], wherein the mesothelioma is pleural mesothelioma and/or peritoneal mesothelioma.

[4] The therapeutic agent according to [1], wherein the mesothelioma is pleural mesothelioma.

[5] A therapeutic agent according to any one of [1] to [4], wherein the anti-B7-H3 antibody is an antibody comprising a heavy chain comprising CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3, and a light chain comprising CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

[6] The therapeutic agent according to any one of [1] to [4], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a

heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, or an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

[7] The therapeutic agent according to [6], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

[8] The therapeutic agent according to any one of [1] to [4], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or an antibody consisting of a heavy chain consisting of an

amino acid sequence consisting of amino acid residues 20 to 471 of SEQ ID NO: 7 and a light chain consisting of an amino acid sequence consisting of amino acid residues 21 to 233 of SEQ ID NO: 4.

[9] The therapeutic agent according to [8], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[10] The therapeutic agent according to any one of [1] to [9], wherein a lysine residue at the carboxyl terminus of the heavy chain of the anti-B7-H3 antibody is deleted.

[11] The therapeutic agent according to any one of [1] to [10], wherein the average number of units of the drug-linker conjugated per antibody molecule in the anti-B7-H3 antibody-drug conjugate is in the range of from 3.5 to 4.5.

[12] A therapeutic agent for mesothelioma comprising, as an active component, an anti-B7-H3 antibody-drug conjugate represented by the following formula:

[Formula 2]

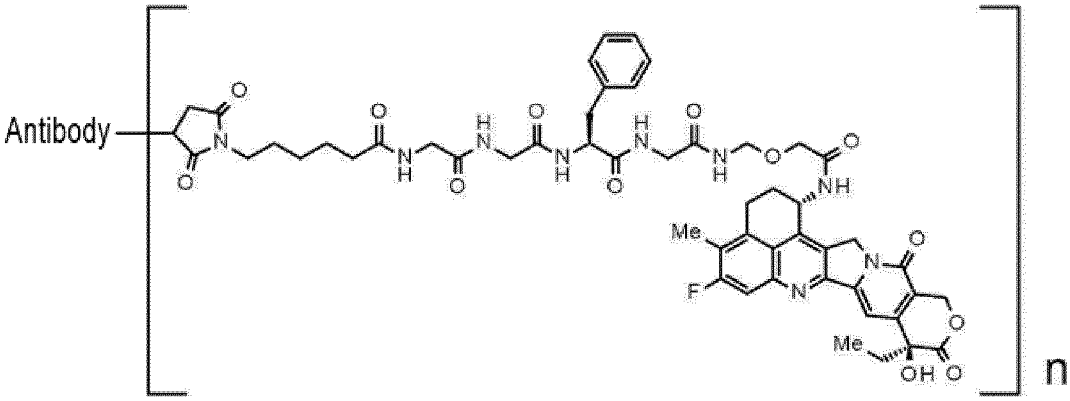

wherein a drug-linker is conjugated to an antibody via a thioether bond, the antibody represents an anti-B7-H3 antibody, and n represents the average number of units of the drug-linker conjugated per antibody molecule.

[13] The therapeutic agent according to [12], wherein the mesothelioma is at least one selected from the group consisting of pleural mesothelioma, peritoneal mesothelioma, pericardial mesothelioma, and tunica vaginalis testis mesothelioma.

[14] The therapeutic agent according to [12], wherein the mesothelioma is pleural mesothelioma and/or peritoneal mesothelioma.

[15] The therapeutic agent according to [12], wherein the mesothelioma is pleural mesothelioma.

[16] The therapeutic agent according to any one of [12] to [15], wherein the anti-B7-H3 antibody is an antibody comprising a heavy chain comprising CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3, and a light chain comprising CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

[17] The therapeutic agent according to any one of [12] to [15], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid

acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, or an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

[18] The therapeutic agent according to [17], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

[19] The therapeutic agent according to any one of [12] to [15], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

[20] The therapeutic agent according to [19], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[21] The therapeutic agent according to any one of [12] to [20], wherein a lysine residue at the carboxyl terminus of the heavy chain of the anti-B7-H3 antibody is deleted.

[22] The therapeutic agent according to any one of [12] to [21], wherein the average number of units of the drug-linker conjugated per antibody molecule in the anti-B7-H3 antibody-drug conjugate is in the range of from 3.5 to 4.5.

[23] A method of treatment for mesothelioma, comprising administering an anti-B7-H3 antibody-drug conjugate in

which a drug-linker represented by the following formula:

[Formula 3]

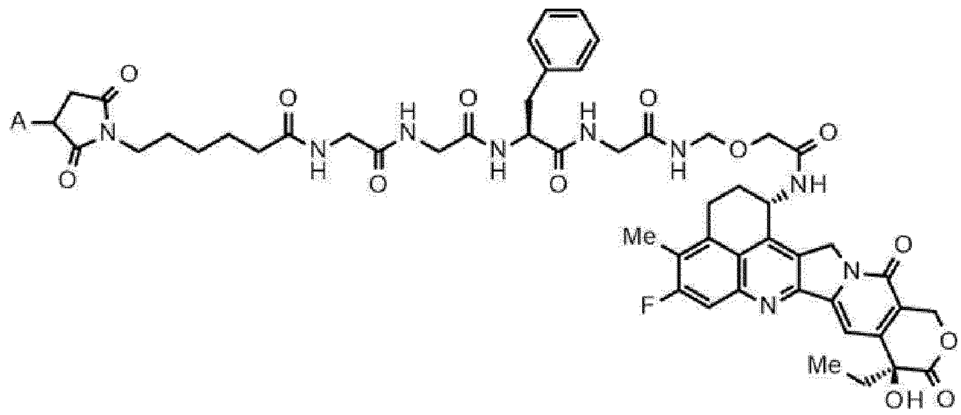

wherein A represents a connecting position to an anti-B7-H3 antibody, is conjugated to the anti-B7-H3 antibody via a thioether bond; to a subject in need of treatment for mesothelioma.

[24] The method of treatment according to [23], wherein the mesothelioma is at least one selected from the group consisting of pleural mesothelioma, peritoneal mesothelioma, pericardial mesothelioma, and tunica vaginalis testis mesothelioma.

[25] The method of treatment according to [23], wherein the mesothelioma is pleural mesothelioma and/or peritoneal mesothelioma.

[26] The method of treatment according to [23], wherein the mesothelioma is pleural mesothelioma.

[27] The method of treatment according to any one of [23] to [26], wherein the anti-B7-H3 antibody is an antibody comprising a heavy chain comprising CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3, and a light chain comprising CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

[28] The method of treatment according to any one of [23] to [26], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino

acid residues 20 to 141 of SEQ ID NO: 7 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, or an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

[29] The method of treatment according to [28], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

[30] The method of treatment according to any one of [23] to [26], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

[31] The method of treatment according to [30], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[32] The method of treatment according to any one of [23] to [31], wherein a lysine residue at the carboxyl terminus of the heavy chain of the anti-B7-H3 antibody is deleted.

[33] The method of treatment according to any one of [23] to [32], wherein the average number of units of the drug-linker conjugated per antibody molecule in the anti-B7-H3 antibody-drug conjugate is in the range of from 3.5 to 4.5.

[34] A method of treatment for mesothelioma, comprising administering an anti-B7-H3 antibody-drug conjugate represented by the following formula:

[Formula 4]

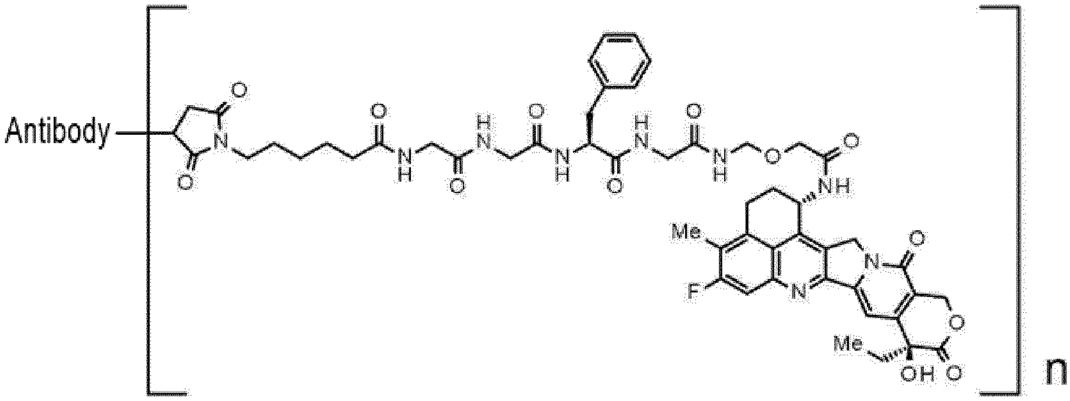

wherein a drug-linker is conjugated to an antibody via a thioether bond, the antibody represents an anti-B7-H3 antibody, and n represents the average number of units of the drug-linker conjugated per antibody molecule; to a subject in need of treatment for mesothelioma.

[35] The method of treatment according to [34], wherein the mesothelioma is at least one selected from the group consisting of pleural mesothelioma, peritoneal mesothelioma, pericardial mesothelioma, and tunica vaginalis testis mesothelioma.

[36] The method of treatment according to [34], wherein the mesothelioma is pleural mesothelioma and/or peritoneal mesothelioma.

[37] The method of treatment according to [34], wherein the mesothelioma is pleural mesothelioma.

[38] The method of treatment according to any one of [34] to [37], wherein the anti-B7-H3 antibody is an antibody comprising a heavy chain comprising CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3, and a light chain comprising CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

[39] The method of treatment according to any one of [34] to [37], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 8, an antibody consisting of a

heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, or an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

[40] The method of treatment according to [39], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

[41] The method of treatment according to any one of [34] to [37], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

[42] The method of treatment according to [41], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[43] The method of treatment according to any one of [34] to [42], wherein a lysine residue at the carboxyl terminus of the heavy chain of the anti-B7-H3 antibody is deleted.

[44] The method of treatment according to any one of [34] to [43], wherein the average number of units of the drug-linker conjugated per antibody molecule in the anti-B7-H3 antibody-drug conjugate is in the range of from 3.5 to 4.5.

[45] An anti-B7-H3 antibody-drug conjugate, for use in treating mesothelioma, in which a drug-linker represented by the following formula:

[Formula 5]

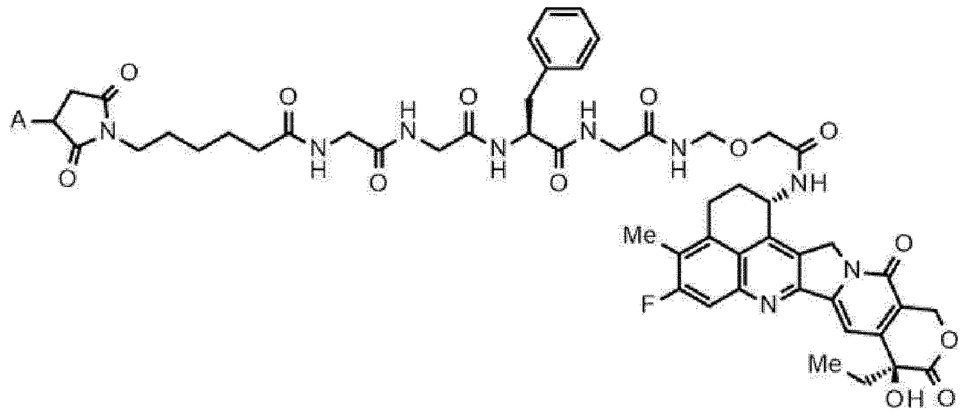

wherein A represents a connecting position to an anti-B7-H3 antibody;
is conjugated to the anti-B7-H3 antibody via a thioether bond.

[46] The anti-B7-H3 antibody-drug conjugate according to [45], wherein the mesothelioma is at least one selected from the group consisting of pleural mesothelioma, peritoneal mesothelioma, pericardial mesothelioma, and tunica vaginalis testis mesothelioma.
[47] The anti-B7-H3 antibody-drug conjugate according to [45], wherein the mesothelioma is pleural mesothelioma and/or peritoneal mesothelioma.
[48] The anti-B7-H3 antibody-drug conjugate according to [45], wherein the mesothelioma is pleural mesothelioma.
[49] The anti-B7-H3 antibody-drug conjugate according to any one of [45] to [48], wherein the anti-B7-H3 antibody is an antibody comprising a heavy chain comprising CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3, and a light chain comprising CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.
[50] The anti-B7-H3 antibody-drug conjugate according to any one of [45] to [48], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino acid residues 20 to 141 of SEQ ID NO: 7 and a light chain comprising a light chain

variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, or an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

[51] The anti-B7-H3 antibody-drug conjugate according to [50], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

[52] The anti-B7-H3 antibody-drug conjugate according to any one of [45] to [48], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

[53] The anti-B7-H3 antibody-drug conjugate according to [52], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[54] The anti-B7-H3 antibody-drug conjugate according to any one of [45] to [53], wherein a lysine residue at the carboxyl terminus of the heavy chain of the anti-B7-H3 antibody is deleted.

[55] The anti-B7-H3 antibody-drug conjugate according to any one of [45] to [54], wherein the average number of units of the drug-linker conjugated per antibody molecule in the anti-B7-H3 antibody-drug conjugate is in the range of from 3.5 to 4.5.

[56] An anti-B7-H3 antibody-drug conjugate, for use in treating mesothelioma, represented by the following formula:

[Formula 6]

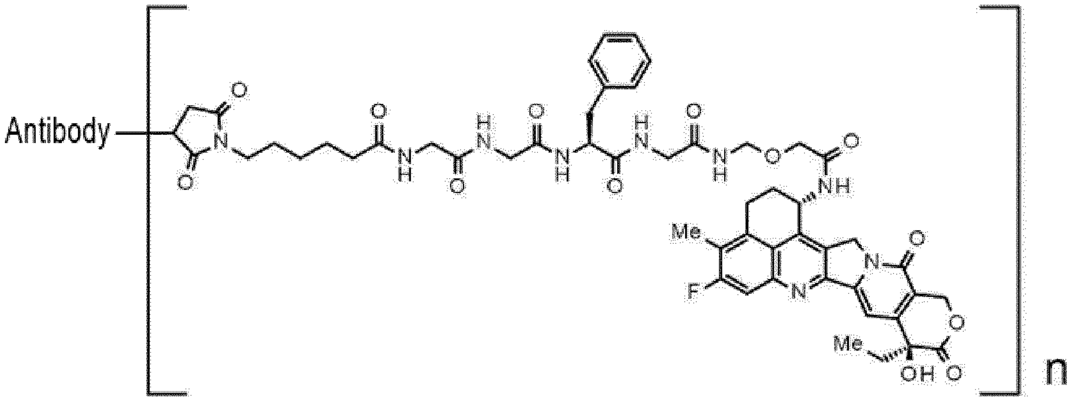

wherein a drug-linker is conjugated to an antibody via a thioether bond, the antibody represents an anti-B7-H3 antibody, and n represents the average number of units of the drug-linker conjugated per antibody molecule.

[57] The anti-B7-H3 antibody-drug conjugate according to [56], wherein the mesothelioma is at least one selected from the group consisting of pleural mesothelioma, peritoneal mesothelioma, pericardial mesothelioma, and tunica vaginalis testis mesothelioma.

[58] The anti-B7-H3 antibody-drug conjugate according to [56], wherein the mesothelioma is pleural mesothelioma and/or peritoneal mesothelioma.

[59] The anti-B7-H3 antibody-drug conjugate according to [56], wherein the mesothelioma is pleural mesothelioma.

[60] The anti-B7-H3 antibody-drug conjugate according to any one of [56] to [59], wherein the anti-B7-H3 antibody is an antibody comprising a heavy chain comprising CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3 and a light chain comprising CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

[61] The anti-B7-H3 antibody-drug conjugate according to any one of [56] to [59], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid

sequence consisting of amino acid residues 20 to 141 of SEQ ID NO: 7 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, or an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

[62] The anti-B7-H3 antibody-drug conjugate according to [61], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

[63] The anti-B7-H3 antibody-drug conjugate according to any one of [56] to [59], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

[64] The anti-B7-H3 antibody-drug conjugate according to [63], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[65] The anti-B7-H3 antibody-drug conjugate according to any one of [56] to [64], wherein a lysine residue at the carboxyl terminus of the heavy chain of the anti-B7-H3 antibody is deleted.

[66] The anti-B7-H3 antibody-drug conjugate according to any one of [56] to [65], wherein the average number of units of the drug-linker conjugated per antibody molecule in the anti-B7-H3 antibody-drug conjugate is in the range of from 3.5 to 4.5.

[67] Use of an anti-B7-H3 antibody-drug conjugate for the manufacture of a medicament for treating mesothelioma, in which a drug-linker represented by the following formula:

[Formula 7]

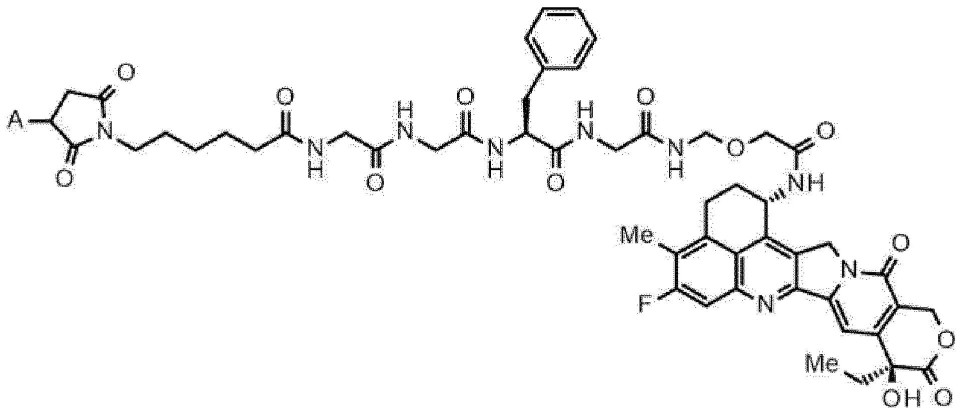

wherein A represents a connecting position to an anti-B7-H3 antibody; is conjugated to the anti-B7-H3 antibody via a thioether bond.

[68] The use according to [67], wherein the mesothelioma is at least one selected from the group consisting of pleural mesothelioma, peritoneal mesothelioma, pericardial mesothelioma, and tunica vaginalis testis mesothelioma.

[69] The use according to [67], wherein the mesothelioma is pleural mesothelioma and/or peritoneal mesothelioma.

[70] The use according to [67], wherein the mesothelioma is pleural mesothelioma.

[71] The use according to any one of [67] to [70], wherein the anti-B7-H3 antibody is an antibody comprising a heavy chain comprising CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3 and a light chain comprising CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

[72] The use according to any one of [67] to [70], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 7 and a light chain comprising a light chain variable region consisting of an

amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, or an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

[73] The use according to [72], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

[74] The use according to any one of [67] to [70], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

[75] The use according to [74], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[76] The use according to any one of [67] to [75], wherein a lysine residue at the carboxyl terminus of the heavy chain of the anti-B7-H3 antibody is deleted.

[77] The use according to any one of [67] to [76], wherein the average number of units of the drug-linker conjugated per antibody molecule in the anti-B7-H3 antibody-drug conjugate is in the range of from 3.5 to 4.5.

[78] Use of an anti-B7-H3 antibody-drug conjugate for the manufacture of a medicament for treating mesothelioma, in which the anti-B7-H3 antibody-drug conjugate is represented by the following formula:

[Formula 8]

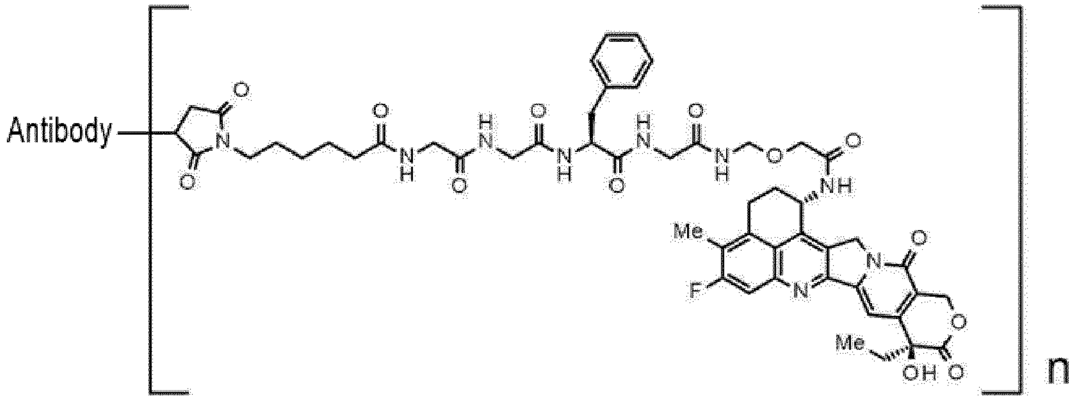

wherein a drug-linker is conjugated to an antibody via a thioether bond, the antibody represents an anti-B7-H3 antibody, and n represents the average number of units of the drug-linker conjugated per antibody molecule.

[79] The use according to [78], wherein the mesothelioma is at least one selected from the group consisting of pleural mesothelioma, peritoneal mesothelioma, pericardial mesothelioma, and tunica vaginalis testis mesothelioma.

[80] The use according to [78], wherein the mesothelioma is pleural mesothelioma and/or peritoneal mesothelioma.

[81] The use according to [78], wherein the mesothelioma is pleural mesothelioma.

[82] The use according to any one of [78] to [81], wherein the anti-B7-H3 antibody is an antibody comprising a heavy chain comprising CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3, and a light chain comprising CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

[83] The use according to any one of [78] to [81], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 9, an antibody consisting of a

heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, or an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

[84] The use according to [83], wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

[85] The use according to any one of [78] to [81], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

[86] The use according to [85], wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

[87] The use according to any one of [78] to [86], wherein a lysine residue at the carboxyl terminus of the heavy chain of the anti-B7-H3 antibody is deleted.

[88] The use according to any one of [78] to [87], wherein the average number of units of the drug-linker conjugated per antibody molecule in the anti-B7-H3 antibody-drug conjugate is in the range of from 3.5 to 4.5.

## Advantageous Effects of Invention

The present invention provides a therapeutic agent for mesothelioma comprising an anti-B7-H3 antibody-drug conjugate, and/or a method of treatment for mesothelioma comprising administering an anti-B7-H3 antibody-drug conjugate to a subject.

## Brief Description of Drawings

[Figure 1] Figure 1 is a diagram showing the amino acid sequence of B7-H3 variant 1 (SEQ ID NO: 1).

[Figure 2] Figure 2 is a diagram showing the amino acid sequence of B7-H3 variant 2 (SEQ ID NO: 2).

[Figure 3] Figure 3 is a diagram showing the amino acid sequence of a heavy chain of an anti-B7-H3 antibody (M30-H1 type) (SEQ ID NO: 3).

[Figure 4] Figure 4 is a diagram showing the amino acid sequence of a light chain of an anti-B7-H3 antibody (M30-L4 type) (SEQ ID NO: 4).

[Figure 5] Figure 5 is a diagram showing the antitumor effects of an anti-B7-H3 antibody-drug conjugate (1) in mice inoculated with human mesothelioma cell line MSTO-211H cells. White diamonds represent a 10 mM acetate buffer (pH 5.5) and 5% sorbitol (ABS buffer) treated group, white circles represent a 3 mg/kg anti-B7-H3 antibody-drug conjugate (1) treated group, and black triangles represent a 10 mg/kg anti-B7-H3 antibody-drug conjugate (1) treated group.

[Figure 6] Figure 6 is a diagram showing the antitumor effects of an anti-B7-H3 antibody-drug conjugate (1) in a mesothelioma PDX model. Mice implanted with tumor fragments derived from a patient with mesothelioma were treated with ABS buffer (black circle) or 10 mg/kg anti-B7-H3 antibody-drug conjugate (1) (white circle).

[Figure 7] Figure 7 is a diagram showing the amino acid sequence of a heavy chain of an anti-B7-H3 antibody (M30-H2 type) (SEQ ID NO: 5).

[Figure 8] Figure 8 is a diagram showing the amino acid sequence of a heavy chain of an anti-B7-H3 antibody (M30-H3 type) (SEQ ID NO: 6).

[Figure 9] Figure 9 is a diagram showing the amino acid sequence of a heavy chain of an anti-B7-H3 antibody (M30-H4 type) (SEQ ID NO: 7).

[Figure 10] Figure 10 is a diagram showing the amino acid sequence of a light chain of an anti-B7-H3 antibody (M30-L1 type) (SEQ ID NO: 8).

[Figure 11] Figure 11 is a diagram showing the amino acid sequence of a light chain of an anti-B7-H3 antibody (M30-L2 type) (SEQ ID NO: 9).

[Figure 12] Figure 12 is a diagram showing the amino acid sequence of a light chain of an anti-B7-H3 antibody (M30-L3 type) (SEQ ID NO: 10).

[Figure 13] Figure 13 is a diagram showing the amino acid sequence of a light chain of an anti-B7-H3 antibody (M30-L5 type) (SEQ ID NO: 11).

[Figure 14] Figure 14 is a diagram showing the amino acid sequence of a light chain of an anti-B7-H3 antibody (M30-L6 type) (SEQ ID NO: 12).

[Figure 15] Figure 15 is a diagram showing the amino acid sequence of a light chain of an anti-B7-H3 antibody (M30-L7 type) (SEQ ID NO: 13).

## Description of Embodiments

Hereinafter, preferred modes for carrying out the present invention are described. The embodiments described below are given merely for illustrating one example of a typical embodiment of the present invention and are not intended to limit the scope of the present invention.

### 1. Mesothelioma

In the present invention, the term "mesothelioma" or "malignant mesothelioma" means a malignant tumor developed from mesothelia originated from the mesoderm (pleura, peritoneum, pericardium, and the like). Mesotheliomas are, depending on origin site, divided into pleural mesothelioma, peritoneal mesothelioma, pericardial mesothelioma, and the like. Mesotheliomas are classified into epithelial type, sarcoma type, and biphasic type based on histological diagnosis of tumor cells.

As the symptoms of malignant pleural mesothelioma, for example, chest pain, cough, difficulty breathing and chest tightness due to a large amount of pleural fluid can be exemplified. It is characteristic of malignant peritoneal mesothelioma to show no symptoms at an early stage. As symptoms of progressed malignant peritoneal mesothelioma, for example, abdominal bloating due to accumulated ascites, abdominal pain, lower back pain, loss of appetite, abnormal defecation, and a lump in the abdomen can be exemplified.

As examination methods for mesotheliomas, for example, imaging examination, cytology, and biopsy using CT (Computed Tomography), PET (Positron Emission Tomography), MRI (Magnetic Resonance Imaging), and the like can be exemplified. For confirmation of a diagnosis of pleural mesothelioma, it is desirable to carry out a reliable histological diagnosis by a pleural biopsy.

Examples of mesothelioma include pleural mesothelioma, peritoneal mesothelioma, pericardial mesothelioma, tunica vaginalis testis mesothelioma and the like. Among these, pleural mesothelioma is particularly found at high percentages.

### 2. Anti-B7-H3 antibody-drug conjugate

The anti-B7-H3 antibody-drug conjugate used in the present invention is an anti-B7-H3 antibody-drug conjugate

in which a drug-linker represented by the following formula:

[Formula 9]

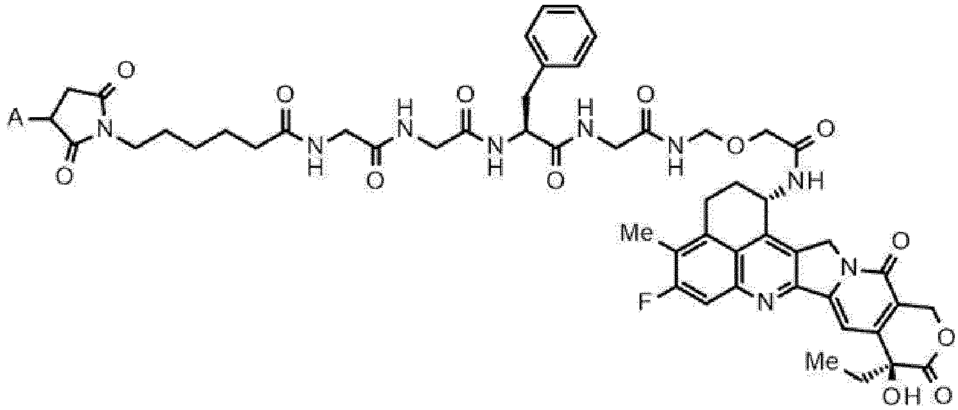

wherein A represents a connecting position to an anti-B7-H3 antibody;
is conjugated to the anti-B7-H3 antibody via a thioether bond.

In the present invention, the partial structure consisting of a linker and a drug in the anti-B7-H3 antibody-drug conjugate is referred to as a "drug-linker". The drug-linker is connected to a thiol group (in other words, the sulfur atom of a cysteine residue) formed at an interchain disulfide bond site (two sites between heavy chains, and two sites between a heavy chain and a light chain) in the antibody.

The drug-linker of the present invention includes exatecan (IUPAC name: (1S,9S)-1-amino-9-ethyl-5-fluoro-1,2,3,9,12,15-hexahydro-9-hydroxy-4-methyl-10H, 13H-benzo[de]pyrano[3',4':6,7]indolizino[1,2-b]quinolin-10,13-dione, (also expressed as chemical name: (1S,9S)-1-amino-9-ethyl-5-fluoro-2,3-dihydro-9-hydroxy-4-methyl-1H,12H-benzo[de]pyrano[3',4' :6,7]indolizino[1,2-b]quinolin-10,13(9H,15H)-dione)), which is a topoisomerase I inhibitor, as a component. Exatecan is a camptothecin derivative having an antitumor effect, represented by the following formula:

[Formula 10]

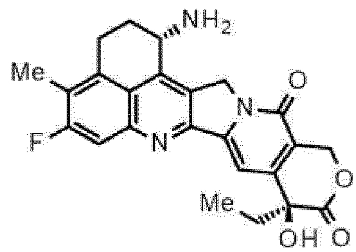

The anti-B7-H3 antibody-drug conjugate used in the present invention can also be represented by the following formula:

[Formula 11]

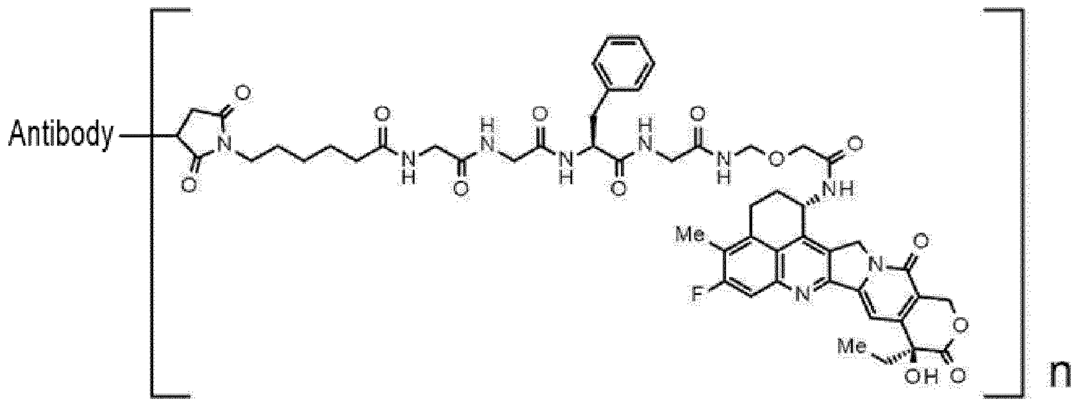

wherein, the drug-linker is conjugated to an anti-B7-H3 antibody via a thioether bond. The meaning of n is the same as the so-called average number of conjugated drug molecules (DAR; Drug-to-Antibody Ratio), and indicates the average number of units of the drug-linker conjugated per antibody molecule.

After migrating into cancer cells, the anti-B7-H3 antibody-drug conjugate used in the present invention releases the compound represented by the following formula:

[Formula 12]

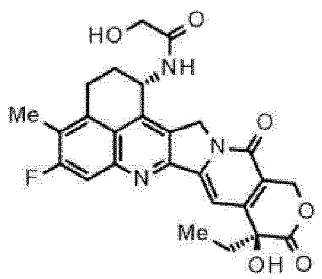

and thereby exerts an antitumor effect.

The aforementioned compound is inferred to be the original source of the antitumor activity of the anti-B7-H3 antibody-drug conjugate used in the present invention, and is presumed to have a topoisomerase I inhibitory effect (Ogitani Y. et al., Clinical Cancer Research, 2016, Oct 15; 22(20):5097-5108, Epub 2016 Mar 29).

The anti-B7-H3 antibody-drug conjugate used in the present invention is also presumed to have a bystander effect (Ogitani Y. et al., Cancer Science (2016) 107, 1039-1046) .

The bystander effect is considered to be exerted through a process such that the anti-B7-H3 antibody-drug conjugate used in the present invention is internalized in cancer cells expressing the target and the aforementioned compound is released and then exerts an antitumor effect also on nearby cancer cells not expressing the target.

3. Anti-B7-H3 antibody

B7-H3, one of the B7 family members expressed in antigen-presenting cells as a co-stimulator, is considered to act on receptors on T cells and enhance or suppress immune effect.

B7-H3 is a single transmembrane protein and has two variants. B7-H3 variant 1 (4Ig-B7-H3) contains two each of V- or C-like Ig domains, and B7-H3 variant 2 (2Ig-B7-H3) contains one each of V- or C-like Ig domain.

The B7-H3 used in the present invention can be used by direct purification from B7-H3-expressing cells of human and non-human mammals (a rat, a mouse, and the like), or can be used by preparing cell membrane fractions of the aforementioned cells, or B7-H3 is synthesized in vitro, or can be obtained by genetically engineering host cells to produce it. In the genetic engineering, specifically, B7-H3 cDNAs are integrated into vectors that permit expression and then synthesized in a solution containing enzymes, substrates, and energy materials required for the transcription and translation, or a host cell of other prokaryotes or eukaryotes is transformed to express B7-H3 thereby obtaining the protein.

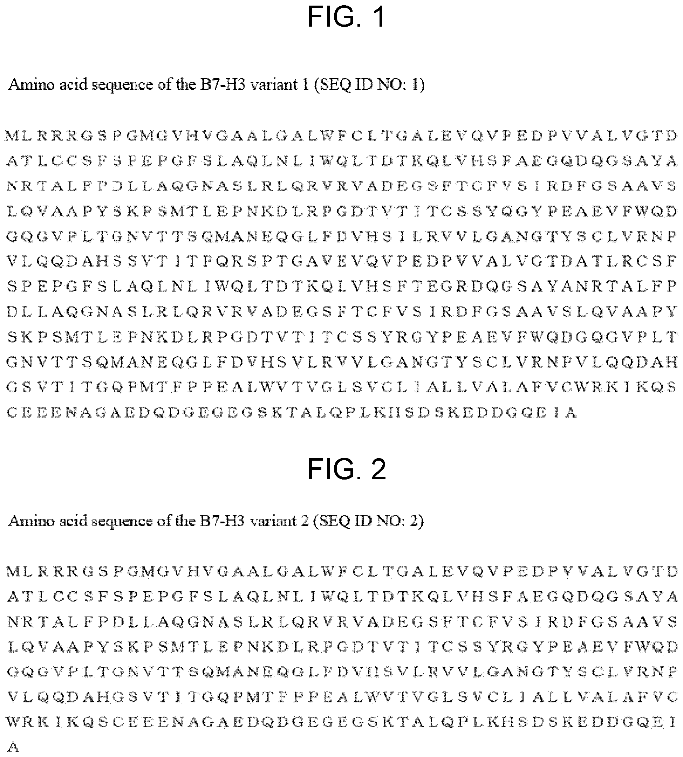

The amino acid sequence of the open reading frame (ORF) of the human B7-H3 variant 1 gene is represented by SEQ ID NO: 1 of the Sequence Listing. The sequence of SEQ ID NO: 1 is shown in Figure 1.

The amino acid sequence of the ORF of the human B7-H3 variant 2 gene is represented by SEQ ID NO: 2 of

the Sequence Listing. The sequence of SEQ ID NO: 2 is shown in Figure 2.

In each of the above amino acid sequences of B7-H3, proteins consisting of an amino acid sequence in which one or several amino acids are substituted, deleted and/or added and having equivalent biological activities to the above protein are also included in B7-H3.

Mature human B7-H3 variant 1 from which a signal sequence is removed corresponds to the amino acid sequence consisting of amino acid residues 27 to 534 of the amino acid sequence represented by SEQ ID NO: 1. Mature human B7-H3 variant 2 from which a signal sequence is removed corresponds to the amino acid sequence consisting of amino acid residues 27 to 316 of the amino acid sequence represented by SEQ ID NO: 2.

The anti-B7-H3 antibody in the anti-B7-H3 antibody-drug conjugate used in the present invention may be derived from any species, but is preferably an antibody derived from a human, a rat, a mouse, or a rabbit. In cases when the antibody is derived from species other than human species, it is preferably chimerized or humanized using a well-known technique. The antibody of the present invention may be a polyclonal antibody or a monoclonal antibody and is preferably a monoclonal antibody.

The anti-B7-H3 antibody in the anti-B7-H3 antibody-drug conjugate used in the present invention is an antibody preferably having the characteristic of being able to target cancer cells, and is preferably an antibody possessing the property of being able to recognize a cancer cell, the property of being able to bind to a cancer cell, the property of being incorporated and internalized in a cancer cell, and/or cytocidal activity against cancer cells.

The binding activity of the antibody against cancer cells can be confirmed using flow cytometry. The internalization of the antibody into cancer cells can be confirmed using (1) an assay of visualizing an antibody incorporated in cells under a fluorescence microscope using a secondary antibody (fluorescently labeled) binding to the therapeutic antibody (Cell Death and Differentiation (2008) 15, 751-761), (2) an assay of measuring a fluorescence intensity incorporated in cells using a secondary antibody (fluorescently labeled) binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant complex protein of a diphtheria toxin catalytic domain and protein G may be used.

The antitumor activity of the antibody can be confirmed in vitro by determining inhibitory activity against cell growth. For example, a cancer cell line overexpressing a target protein for the antibody is cultured, and the antibody is added at varying concentrations into the culture system in order to determine inhibitory activity against focus formation, colony formation, and spheroid growth. The antitumor activity can be confirmed in vivo, for example, by administering the antibody to nude mice inoculated with a cancer cell line highly expressing the target protein, and determining changes in the cancer cells.

Since the compound conjugated in the anti-B7-H3 antibody-drug conjugate exerts an antitumor effect, it is preferred but not essential that the anti-B7-H3 antibody itself should have an antitumor effect. For the purpose of specifically and selectively exerting the cytotoxic activity of the antitumor compound against cancer cells, it is important and also preferred that the antibody should have the property of being internalized and migrating into cancer cells.

The anti-B7-H3 antibody in the anti-B7-H3 antibody-drug conjugate used in the present invention can be obtained by a procedure known in the art. For example, the antibody of the present invention can be obtained using a method usually carried out in the art, which involves immunizing animals with an antigenic polypeptide and collecting and purifying antibodies produced in vivo. The origin of the antigen is not limited to humans, and the animals may be immunized with an antigen derived from a non-human animal such as a mouse, a rat and the like. In this case, the cross-reactivity of antibodies binding to the obtained heterologous antigen with human antigens can be tested to screen for an antibody applicable to a human disease.

Alternatively, antibody-producing cells which produce antibodies against the antigen can be fused with myeloma cells according to a method known in the art (e.g., Kohler and Milstein, Nature (1975) 256, p. 495-497; Kennet, R. ed., Monoclonal Antibodies, p. 365-367, Plenum Press, N.Y. (1980)) to establish hybridomas, from which monoclonal antibodies can in turn be obtained.

The antigen can be obtained by genetically engineering host cells to produce a gene encoding the antigenic protein. Specifically, vectors that permit expression of the antigen gene are prepared and transferred to host cells so that the gene is expressed. The antigen thus expressed can be purified. The antibody can also be obtained by a method of immunizing animals with the above-described genetically engineered antigen-expressing cells or a cell line expressing the antigen.

The anti-B7-H3 antibody in the anti-B7-H3 antibody-drug conjugate used in the present invention is preferably a recombinant antibody obtained by artificial modification for the purpose of decreasing heterologous antigenicity to humans such as a chimeric antibody or a humanized antibody, or is preferably an antibody having only the gene sequence of an antibody derived from a human, that is, a human antibody. These antibodies can be produced using a known method.

As the chimeric antibody, an antibody in which antibody variable and constant regions are derived from different species, for example, a chimeric antibody in which a mouse- or rat-derived antibody variable region is connected to a

human-derived antibody constant region can be exemplified (Proc. Natl. Acad. Sci. USA, 81, 6851-6855, (1984)).

As the humanized antibody, an antibody obtained by integrating only the complementarity determining region (CDR) of a heterologous antibody into a human-derived antibody (Nature (1986) 321, pp. 522-525), an antibody obtained by grafting a part of the amino acid residues of the framework of a heterologous antibody as well as the CDR sequence of the heterologous antibody to a human antibody by a CDR-grafting method (WO 90/07861), and an antibody humanized using a gene conversion mutagenesis strategy (U.S. Patent No. 5821337) can be exemplified.

As the human antibody, an antibody generated by using a human antibody-producing mouse having a human chromosome fragment including genes of a heavy chain and a light chain of a human antibody (see Tomizuka, K. et al., Nature Genetics (1997) 16, p.133-143; Kuroiwa, Y. et. al., Nucl. Acids Res. (1998) 26, p.3447-3448; Yoshida, H. et. al., Animal Cell Technology: Basic and Applied Aspects vol.10, p.69-73 (Kitagawa, Y., Matsuda, T. and Iijima, S. eds.), Kluwer Academic Publishers, 1999; Tomizuka, K. et. al., Proc. Natl. Acad. Sci. USA (2000) 97, p.722-727, etc.) can be exemplified. As an alternative, an antibody obtained by phage display, the antibody being selected from a human antibody library (see Wormstone, I. M. et. al, Investigative Ophthalmology & Visual Science. (2002)43 (7), p.2301-2308; Carmen, S. et. al., Briefings in Functional Genomics and Proteomics (2002), 1(2), p.189-203; Siriwardena, D. et. al., Ophthalmology (2002) 109(3), p.427-431, etc.) can be exemplified.

In the anti-B7-H3 antibody in the anti-B7-H3 antibody-drug conjugate used in present invention, modified variants of the antibody are also included. The modified variant refers to a variant obtained by subjecting the antibody according to the present invention to chemical or biological modification. Examples of the chemically modified variant include variants including a linkage of a chemical moiety to an amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N terminus by being expressed in a prokaryotic host cell. Further, an antibody labeled so as to enable the detection or isolation of the antibody or an antigen according to the present invention, for example, an enzyme-labeled antibody, a fluorescence-labeled antibody, and an affinity-labeled antibody are also included in the meaning of the modified variant. Such a modified variant of the antibody according to the present invention is useful for improving the stability and blood retention of the antibody, reducing the antigenicity thereof, detecting or isolating an antibody or an antigen, and so on.

Further, by regulating the modification of a glycan which is linked to the antibody according to the present invention (glycosylation, defucosylation, etc.), it is possible to enhance antibody-dependent cellular cytotoxic activity. As the technique for regulating the modification of a glycan of antibodies, WO 99/54342, WO 00/61739, WO 02/31140, WO 2007/13385, and WO 2013/120066 etc. are known. However, the technique is not limited thereto. In the antibody according to the present invention, antibodies in which the modification of a glycan is regulated are also included.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (complement activation, antibody-dependent cellular cytotoxicity, etc.) of the antibody. Therefore, in the antibody according to the present invention, antibodies subjected to such modification and functional fragments of the antibody are also included, and deletion variants in which one or two amino acids have been deleted at the carboxyl terminus of the heavy chain, variants obtained by amidation of the deletion variants (for example, a heavy chain in which the carboxyl terminal proline residue has been amidated), and the like are also included. The type of deletion variant having a deletion at the carboxyl terminus of the heavy chain of the antibody according to the present invention is not limited to the above variants as long as the antigen-binding affinity and the effector function are conserved. The two heavy chains constituting the antibody according to the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions; however, an antibody in which one amino acid residue at the carboxyl terminus has been deleted in both of the two heavy chains in the antibody according to the present invention can be preferably exemplified.

As isotypes of the antibody according to the present invention, for example, IgG (IgG1, IgG2, IgG3, IgG4) can be exemplified, and IgG1 or IgG2 can be exemplified preferably.

In the present invention, the term "anti-B7-H3 antibody" refers to an antibody which binds specifically to B7-H3 (B cell antigen #7 homolog 3; PD-L3; CD276), and preferably has an activity of internalization in B7-H3-expressing cells by binding to B7-H3.

Examples of the anti-B7-H3 antibody include any combinations of a heavy chain comprising a heavy chain variable region consisting of any one of (1) the amino acid sequence consisting of amino acid residues 20 to 141 of SEQ

ID NO: 3, 5, 6 or 7 of the Sequence Listing, (2) an amino acid sequence having at least 95% or more homology with the amino acid sequence of the above (1), and (3) an amino acid sequence of the above (1) in which one or several amino acids are deleted, substituted or added, and a light chain comprising a light chain variable region consisting of any one of (4) the amino acid sequence consisting of amino acid residues 21 to 128 of SEQ ID NO: 4, 8, 9, 10, 11, 12 or 13, (5) an amino acid sequence having at least 95% or more homology with the amino acid sequence of the above (4), and (6) an amino acid sequence of the above (4) in which one or several amino acids are deleted, substituted or added, and preferably, M30-H1-L4 (International Publication No. WO 2014/057687) can be exemplified. The term "several" in the present specification means 1 to 10 amino acids, 1 to 9 amino acids, 1 to 8 amino acids, 1 to 7 amino acids, 1 to 6 amino acids, 1 to 5 amino acids, 1 to 4 amino acids, 1 to 3 amino acids, or 1 or 2 amino acids.

For the substitution of amino acids in the present specification, conserved amino acid substitution is preferred. The conserved amino acid substitution is a replacement which occurs within amino acid groups associated with amino acid side chains. Preferable amino acid groups are as follows: acidic group = aspartic acid, glutamic acid; basic group = lysine, arginine, histidine; non-polar group = alanine, valine, leucine, isoleucine, proline, phenylalanine, methionine, tryptophan; and non-charged, polar family = glycine, asparagine, glutamine, cysteine, serine, threonine, and tyrosine. Other preferable amino acid groups are as follows: aliphatic hydroxy group = serine and threonine; amide-containing group = asparagine and glutamine; aliphatic group = alanine, valine, leucine, and isoleucine; and aromatic group = phenylalanine, tryptophan, and tyrosine. Such an amino acid substitution is preferably carried out in the range which does not decrease the properties of the substance having the original amino acid sequence.

Examples of the antibody having a preferable combination of the above heavy chain and light chain include an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, and an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

Examples of the antibody having a further preferable combination include an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino acid sequence consisting of amino acid residues

21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, and an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

Further, other preferable combinations include an antibody consisting of a heavy chain consisting of an amino acid sequence represented by SEQ ID NO: 3 and a light chain consisting of an amino acid sequence represented by SEQ ID NO: 8, an antibody consisting of a heavy chain consisting of an amino acid sequence represented by SEQ ID NO: 3 and a light chain consisting of an amino acid sequence represented by SEQ ID NO: 9, an antibody consisting of a heavy chain consisting of an amino acid sequence represented by SEQ ID NO: 3 and a light chain consisting of an amino acid sequence represented by SEQ ID NO: 10, an antibody consisting of a heavy chain consisting of an amino acid sequence represented by SEQ ID NO: 3 and a light chain consisting of an amino acid sequence represented by SEQ ID NO: 4, an antibody consisting of a heavy chain consisting of an amino acid sequence represented by SEQ ID NO: 3 and a light chain consisting of an amino acid sequence represented by SEQ ID NO: 11, an antibody consisting of a heavy chain consisting of an amino acid sequence represented by SEQ ID NO: 3 and a light chain consisting of an amino acid sequence represented by SEQ ID NO: 12, an antibody consisting of a heavy chain consisting of an amino acid sequence represented by SEQ ID NO: 3 and a light chain consisting of an amino acid sequence represented by SEQ ID NO: 13, an antibody consisting of a heavy chain consisting of an amino acid sequence represented by SEQ ID NO: 7 and a light chain consisting of an amino acid sequence represented by SEQ ID NO: 8, an antibody consisting of a heavy chain consisting of an amino acid sequence represented by SEQ ID NO: 7 and a light chain consisting of an amino acid sequence represented by SEQ ID NO: 9, an antibody consisting of a heavy chain consisting of an amino acid sequence represented by SEQ ID NO: 7 and a light chain consisting of an amino acid sequence represented by SEQ ID NO: 10, and an antibody consisting of a heavy chain consisting of an amino acid sequence represented by SEQ ID NO: 7 and a light chain consisting of an amino acid sequence represented by SEQ ID NO: 4.

By combining sequences having high homologies with the above heavy chain amino acid sequences and light chain amino acid sequences, it is possible to select antibodies having the cellular cytotoxic activity equivalent to each of the aforementioned antibodies. Such a homology is typically 80% or more homology, preferably 90% or more homology, more preferably 95% or more homology, and most preferably 99% or more homology. Alternatively, by combining amino acid sequences in which one to several amino acid residues are substituted, deleted or added in amino acid sequences of the heavy chain or light chain, it is possible to select antibodies having the cellular cytotoxic activity equivalent to each of the aforementioned antibodies.

The homology between two types of amino acid sequences can be determined by using the default parameter of Blast algorithm version 2.2.2 (Altschul, Stephen F., Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res.25:3389-3402). Blast algorithm can also be used by accessing www.ncbi.nlm.nih.gov/blast on the internet.

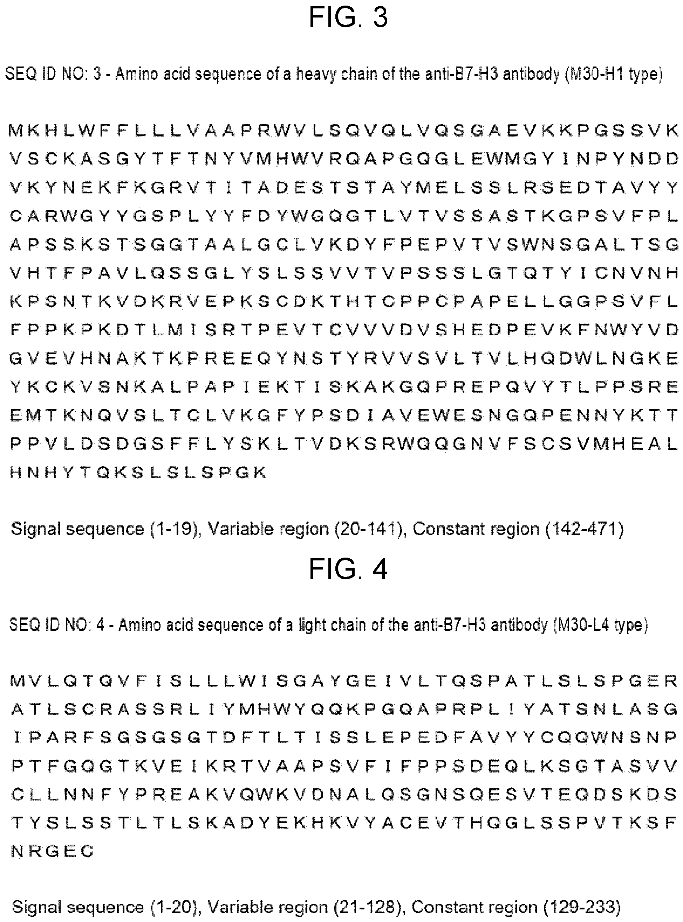

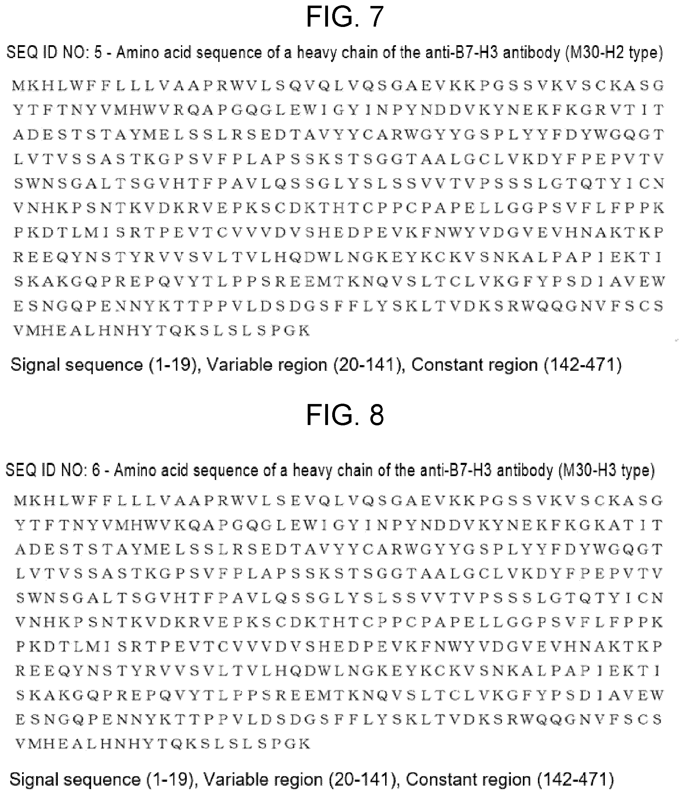

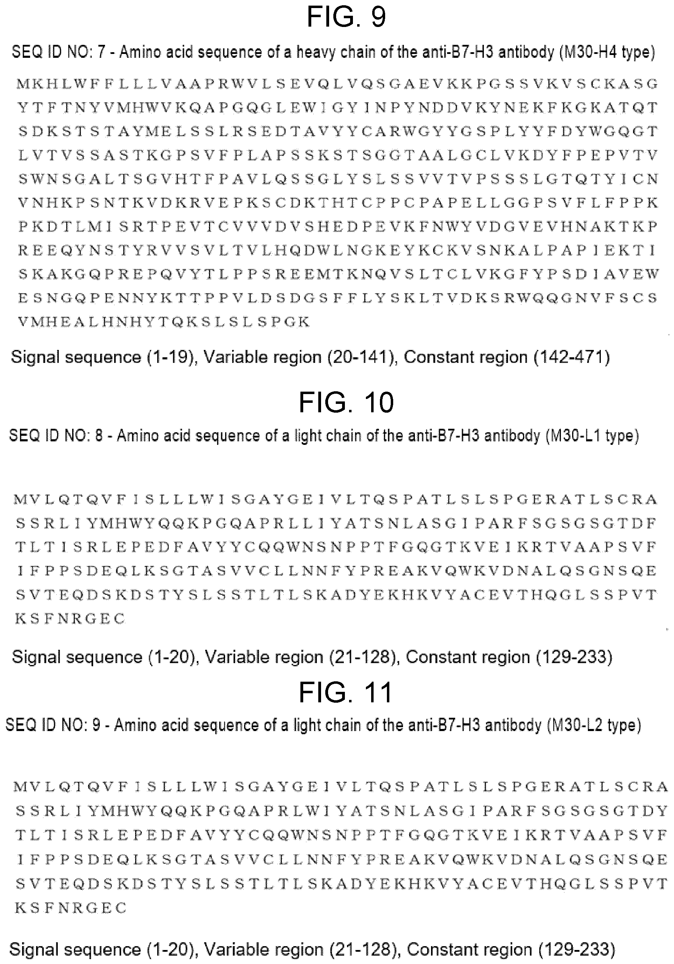

In the heavy chain amino acid sequence represented by SEQ ID NO: 3, 5, 6 or 7 of the Sequence Listing, the amino acid sequence consisting of amino acid residues 1 to 19 is a signal sequence, the amino acid sequence consisting of amino acid residues 20 to 141 is a variable region, and the amino acid sequence consisting of amino acid residues 142 to 471 is a constant region. The sequence of SEQ ID NO: 3 is shown in Figure 3, the sequence of SEQ ID NO: 5 is shown in Figure 7, the sequence of SEQ ID NO: 6 is shown in Figure 8, and the sequence of SEQ ID NO: 7 is shown in Figure 9, respectively.

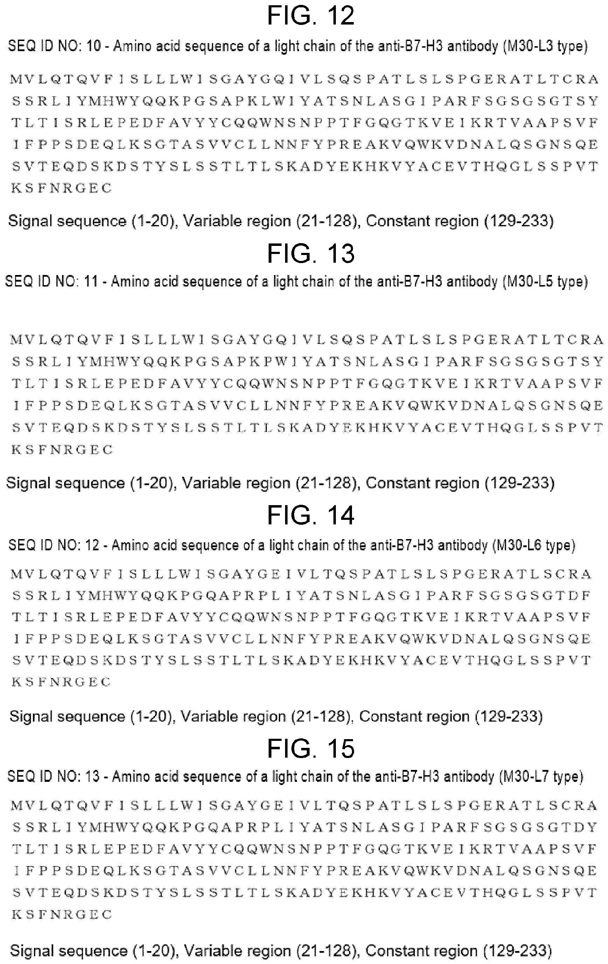

In the light chain amino acid sequence represented by SEQ ID NO: 4, 8, 9, 10, 11, 12 or 13 of the Sequence Listing, the amino acid sequence consisting of amino acid residues 1 to 20 is a signal sequence, the amino acid sequence consisting of amino acid residues 21 to 128 is a variable region, and the amino acid sequence consisting of amino acid

residues 129 to 233 is a constant region. The sequence of SEQ ID NO: 4 is shown in Figure 4, the sequence of SEQ ID NO: 8 is shown in Figure 10, the sequence of SEQ ID NO: 9 is shown in Figure 11, the sequence of SEQ ID NO: 10 is shown in Figure 12, the sequence of SEQ ID NO: 11 is shown in Figure 13, the sequence of SEQ ID NO: 12 is shown in Figure 14, and the sequence of SEQ ID NO: 13 is shown in Figure 15, respectively.

4. Production of anti-B7-H3 antibody-drug conjugate

A drug-linker intermediate for use in the production of the anti-B7-H3 antibody-drug conjugate used in the present invention is represented by the following formula.

[Formula 13]

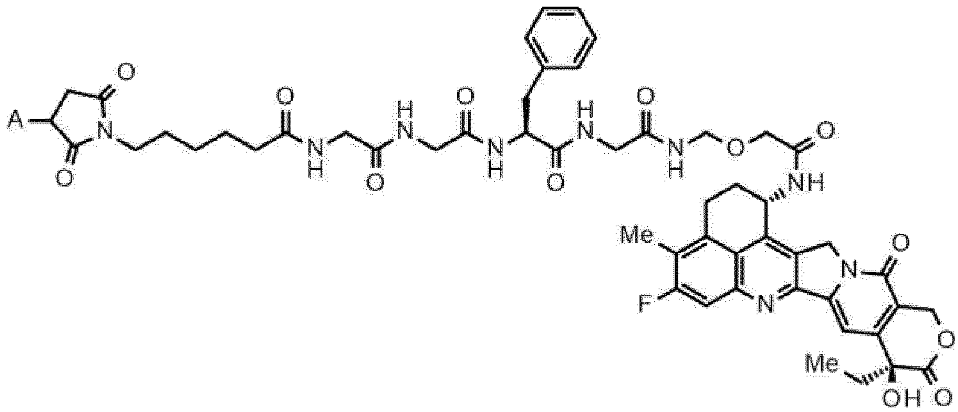

The drug-linker intermediate can be expressed as the chemical name N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide, and can be produced with reference to descriptions in International Publication No. WO 2014/057687, International Publication No. WO 2015/098099, International Publication No. WO 2015/115091, International Publication No. WO 2015/155998, International Publication No. WO 2019/044947, and so on.

The anti-B7-H3 antibody-drug conjugate used in the present invention can be produced by reacting the above-described drug-linker intermediate and an anti-B7-H3 antibody having a thiol group (alternatively referred to as a sulfhydryl group).

The antibody having a sulfhydryl group can be obtained by a method well known in the art (Hermanson, G. T, Bioconjugate Techniques, pp. 56-136, pp. 456-493, Academic Press (1996)). For example, by using 0.3 to 3 molar equivalents of a reducing agent such as tris(2-carboxyethyl)phosphine hydrochloride (TCEP) per interchain disulfide within the antibody and reacting with the antibody in a buffer solution containing a chelating agent such as ethylenediamine tetraacetic acid (EDTA), an antibody having a sulfhydryl group with partially or completely reduced interchain disulfides within the antibody can be obtained.

Further, by using 2 to 20 molar equivalents of the drug-linker intermediate per the antibody having a sulfhydryl group, an antibody-drug conjugate in which 2 to 8 drug molecules are conjugated per antibody molecule can be produced.

The average number of conjugated drug molecules per antibody molecule of the antibody-drug conjugate produced can be determined, for example, by a method of calculation based on measurement of UV absorbance for the anti-B7-H3 antibody-drug conjugate and the conjugation precursor thereof at two wavelengths of 280 nm and 370 nm (UV method), or a method of calculation based on quantification through HPLC measurement for fragments obtained by treating the antibody-drug conjugate with a reducing agent (HPLC method).

Conjugation between the antibody and the drug-linker intermediate and calculation of the average number of conjugated drug molecules per antibody molecule of the antibody-drug conjugate can be performed with reference to descriptions in International Publication No. WO 2014/057687, International Publication No. WO 2017/002776, and so on.

In the present invention, the term "anti-B7-H3 antibody-drug conjugate" refers to an antibody-drug conjugate such that the antibody in the antibody-drug conjugate according to the present invention is an anti-B7-H3 antibody.

The anti-B7-H3 antibody is preferably an antibody comprising a heavy chain comprising CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3, and a light chain comprising CDRL1 consisting

of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4,

more preferably an antibody comprising a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, and

even more preferably an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, or a variant of the antibody in which a lysine residue at the carboxyl terminus of the heavy chain is deleted.

The average number of units of the drug-linker conjugated per antibody molecule in the anti-B7-H3 antibody-drug conjugate is preferably 2 to 8, more preferably 3 to 5, even more preferably 3.5 to 4.5, and even more preferably about 4. In the present invention, the term "about 4" is preferably 3.8 to 4.2, more preferably 3.9 to 4.1, and even more preferably 4.

The anti-B7-H3 antibody-drug conjugate used in the present invention can be produced with reference to descriptions in International Publication No. WO 2014/057687, International Publication No. WO 2017/002776 and so on.

5. Therapeutic agent and/or method of treatment

The therapeutic agent of the present invention comprises the anti-B7-H3 antibody-drug conjugate used in the present invention. Further, the method of treatment of the present invention comprises administering the anti-B7-H3 antibody-drug conjugate used in the present invention. The therapeutic agent and the method of treatment can be used for the treatment of mesothelioma.

The mesotheliomas for which the therapeutic agent and/or method of treatment of the present invention can be used are not particularly limited as long as they are tumors which develop in the mesothelium, but pleural mesothelioma, peritoneal mesothelioma, pericardial mesothelioma, and tunica vaginalis testis mesothelioma can be exemplified, preferably pleural mesothelioma, and peritoneal mesothelioma can be exemplified, and more preferably pleural mesothelioma can be exemplified.

The presence or absence of B7-H3 and other tumor markers, can be checked by, for example, collecting tumor tissues from a cancer patient and subjecting the formalin fixed paraffin embedded specimen (FFPE) to an examination at a gene product (protein) level, such as an immunohistochemistry (IHC) method, flow cytometry, a western blot method, or an examination at a gene transcription level, such as an in situ hybridization method (ISH), a quantitative PCR method (q-PCR), or a microarray analysis; alternatively, it can also be checked by collecting cell-free blood circulating tumor DNA (ctDNA) from a cancer patient and subjecting to an examination which uses a method such as next-generation sequencing (NGS).

The therapeutic agent and method of treatment of the present invention can be preferably used for mammals, and can be more preferably used for humans.

The antitumor effect of the therapeutic agent and method of treatment of the present invention can be confirmed by generating a model in which a mesothelioma cell line is subcutaneously transplanted into a test animal and applying the therapeutic agent or method of treatment of the present invention. For example, in the case of subcutaneously transplanting a mesothelioma cell line into a test animal as the model animal, an estimated tumor volume is calculated by measuring a tumor diameter, and in the case that the estimated tumor volume is diminished as compared with a control group when the therapeutic agent or method of treatment of the present invention is applied, an antitumor effect is recognized.

Alternatively, the antitumor effect of the therapeutic agent and method of treatment of the present invention can also be confirmed by generating a model in which a test animal is transplanted with a biopsy derived from a patient with a mesothelioma (for example, a PDX mesothelioma model) and applying the therapeutic agent or method of treatment of the present invention, and further can also be confirmed by administering the therapeutic agent or applying the method of treatment of the present invention to a patient with a mesothelioma. The measurement of the antitumor effect can be carried out, for example, using CT, PET, and/or MRI, by confirming change in tumor volumes before and after applying the therapeutic agent or method of treatment of the present invention.

In addition, the antitumor effect of the therapeutic agent and method of treatment of the present invention can be confirmed, in a clinical study, with the Response Evaluation Criteria in Solid Tumors (RECIST) evaluation method, WHO's evaluation method, Macdonald's evaluation method, measurement of body weight, and other methods; and can be determined by indicators such as Complete response (CR), Partial response (PR), Progressive disease (PD), Objective

response rate (ORR), Duration of response (DoR), Progression-free survival (PFS), and Overall survival (OS).

The foregoing methods can provide confirmation of superiority in terms of the antitumor effect of the therapeutic agent and method of treatment of the present invention against mesothelioma compared to existing anticancer agents.

The therapeutic agent and method of treatment of the present invention can retard growth of cancer cells, suppress their proliferation, and further can kill cancer cells. These effects can allow cancer patients to be free from symptoms caused by cancer or can achieve an improvement in the QOL of cancer patients and attain a therapeutic effect by sustaining the lives of the cancer patients. Even if the therapeutic agent and method of treatment do not accomplish the killing of cancer cells, they can achieve higher QOL of cancer patients while achieving longer-term survival, by inhibiting or controlling the growth of cancer cells.

The therapeutic agent of the present invention can be expected to exert a therapeutic effect by application as a systemic therapy to patients, and additionally, by local application to cancer tissues.

The therapeutic agent of the present invention can be administered as a pharmaceutical composition containing at least one pharmaceutically suitable ingredient. The pharmaceutically suitable ingredients can be appropriately selected and applied from formulation additives or the like that are generally used in the art, in view of the dosage, the administration concentration or the like of the anti-B7-H3 antibody-drug conjugate used in the present invention. For example, the therapeutic agent of the present invention can be administered as a pharmaceutical composition (hereinafter, referred to as "the pharmaceutical composition of the present invention") containing a buffer such as a histidine buffer, an excipient such as sucrose or trehalose, and a surfactant such as polysorbate 80 or 20. The pharmaceutical composition of the present invention is a pharmaceutical composition containing, preferably,

(i) per 20 mg of the antibody-drug conjugate,
(ii) a 10 mmol histidine buffer,
(iii) 90 mg of sucrose, and
(iv) 0.2 or 0.3 mg of polysorbate 20.

Further, the pH of the above pharmaceutical composition, in which the antibody-drug conjugate is dissolved in water at a concentration of 20 mg/mL, is preferably 5.7 to 6.1, more preferably 5.8 to 6.0, and even more preferably 5.9.

Further, when the histidine buffer is expressed as the contents of L-histidine and L-histidine hydrochloride, the above pharmaceutical composition at a pH of 5.9 can be expressed as a pharmaceutical composition containing, preferably,

(i) per 20 mg of the antibody-drug conjugate,
(ii) 0.65 mg of L-histidine and 1.22 mg of L-histidine hydrochloride hydrate,
(iii) 90 mg of sucrose, and
(iv) 0.2 or 0.3 mg of polysorbate 20.

Further, when expressed as a unit formulation containing 100 mg of the antibody-drug conjugate, the above pharmaceutical composition at a pH of 5.9 can be expressed as a pharmaceutical composition containing,

(i) 100 mg of the antibody-drug conjugate,
(ii) 3.23 mg of L-histidine and 6.12 mg of L-histidine hydrochloride hydrate,
(iii) 450 mg of sucrose, and
(iv) 1.0 or 1.5 mg of polysorbate 20.

Alternatively, when expressed as an aqueous solution in which the concentration of the antibody-drug conjugate is 20 mg/mL, the above pharmaceutical composition can be expressed as a pharmaceutical composition containing,

(i) 20 mg/mL of the antibody-drug conjugate,
(ii) 10 mM of a histidine buffer,
(iii) 9% sucrose,
(iv) 0.02 or 0.03% polysorbate 20, and
(v) water

and having pH of 5.9.

The pharmaceutical composition of the present invention can be preferably used as an injection, can be more preferably used as an aqueous injection or a lyophilized injection, and can be even more preferably used as a lyophilized injection.

In the case that the pharmaceutical composition of the present invention is an aqueous injection, it can be

preferably diluted with a suitable diluent and then given as an intravenous infusion. For the diluent, a dextrose solution, physiological saline, and the like, can be exemplified, and a dextrose solution can be preferably exemplified, and a 5% dextrose solution can be more preferably exemplified.

In the case that the pharmaceutical composition of the present invention is a lyophilized injection, it can be preferably dissolved in water for injection, subsequently a required amount can be diluted with a suitable diluent and then given as an intravenous infusion. For the diluent, a dextrose solution, physiological saline, and the like, can be exemplified, and a dextrose solution can be preferably exemplified, and a 5% dextrose solution can be more preferably exemplified.

Examples of the administration route which may be used to administer the pharmaceutical composition of the present invention include intravenous, intradermal, subcutaneous, intramuscular and intraperitoneal routes, and preferably include an intravenous route.

The anti-B7-H3 antibody-drug conjugate used in the present invention can be administered to a human once at intervals of 1 to 180 days, and can be preferably administered once a week, once every 2 weeks, once every 3 weeks or once every 4 weeks, and can be even more preferably administered once every 3 weeks. Also, the anti-B7-H3 antibody-drug conjugate used in the present invention can be administered at a dose of about 0.001 to 100 mg/kg, and can be preferably administered at a dose of 0.8 mg/kg, 1.6 mg/kg, 3.2 mg/kg, 4.8 mg/kg, 6.4 mg/kg, 8.0 mg/kg, 12.0 mg/kg, or 16.0 mg/kg, and can be even more preferably administered at a dose of 8.0 mg/kg, or 12.0 mg/kg once every 3 weeks.

The therapeutic agent of the present invention can also be administered in combination with a cancer therapeutic agent other than the anti-B7-H3 antibody-drug conjugate used in the present invention, thereby enhancing the antitumor effect. Other cancer therapeutic agents used for such purpose may be administered to a subject simultaneously with, separately from, or subsequently to the therapeutic agent of the present invention, and may be administered while varying the administration interval for each. Such cancer therapeutic agents are not limited as long as they are agents having antitumor activity, and can be exemplified by at least one selected from the group consisting of irinotecan (CPT-11), cisplatin, carboplatin, oxaliplatin, fluorouracil (5-FU), gemcitabine, capecitabine, paclitaxel, docetaxel, doxorubicin, epirubicin, cyclophosphamide, mitomycin C, tegafur-gimeracil-oteracil combination, cetuximab, panitumumab, bevacizumab, ramucirumab, regorafenib, trifluridine-tipiracil combination, gefitinib, erlotinib, afatinib, methotrexate, pemetrexed, tamoxifen, toremifene, fulvestrant, leuprorelin, goserelin, letrozole, anastrozole, progesterone formulation, trastuzumab emtansine, trastuzumab, pertuzumab and lapatinib.

The therapeutic agent of the present invention can also be used in combination with radiotherapy. For example, a cancer patient may receive radiotherapy before and/or after receiving, or simultaneously with, the treatment by the therapeutic agent of the present invention. Examples of the radiotherapy method can include Stereotactic irradiation (STI) and Stereotactic radiosurgery (SRS).

The therapeutic agent of the present invention can also be used as an adjuvant chemotherapy in combination with a surgical procedure. The surgical procedures for pleural mesothelioma include, for example, Pleurectomy/decortication (P/D) by which only the pleura is excised, and Extrapleural pneumonectomy (EPP) by which the pleura and lung are excised together.

[Examples]

The present invention is specifically described in view of the examples shown below. However, the present invention is not limited to these. Further, it is by no means to be interpreted in a limited way.

Example 1: Production of antibody-drug conjugate

With reference to the production method described in International Publication No. WO 2014/057687 with use of a humanized anti-B7-H3 antibody (an antibody comprising a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4), an anti-B7-H3 antibody-drug conjugate in which a drug-linker represented by the following formula:

[Formula 14]

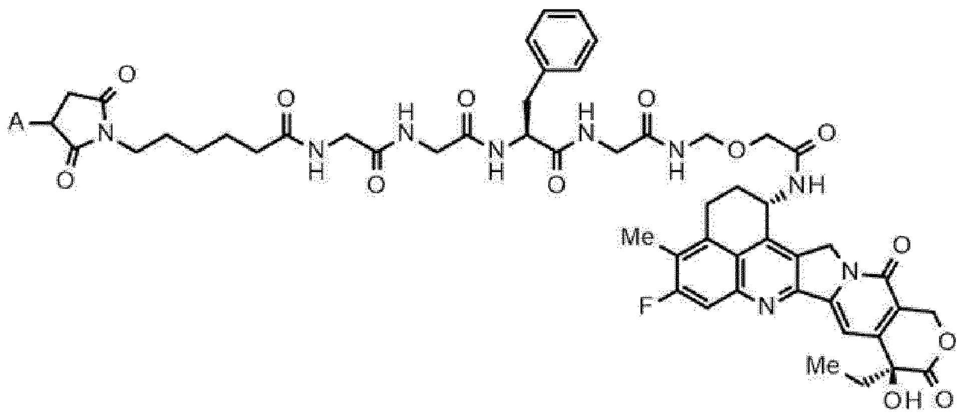

wherein A represents a connecting position to an anti-B7-H3 antibody,
is conjugated to the anti-B7-H3 antibody via a thioether bond (hereinafter, referred to as the "anti-B7-H3 antibody-drug conjugate (1)") was produced. The DAR of the anti-B7-H3 antibody-drug conjugate (1) is 4.0.

Example 2: Antitumor activity of the anti-B7-H3 antibody-drug conjugate (1) on cell line-derived xenograft (CDX) models

Mouse: 5-week-old female BALB/c nude mice (Charles River Laboratories Japan, Inc.) were acclimated for 3 days under SPF conditions before being subjected to the experiment. The mice were fed sterilized solid feed (FR-2, Funabashi Farms Co., Ltd) and given chlorine (sodium hypochlorite)-added tap water.
Measurement, calculation formula: a longer diameter and a shorter diameter of a tumor were measured twice a week using a digital caliper and a tumor volume ($mm^3$) was calculated. The calculation formula is as follows.

$$\text{Tumor volume } (mm^3) = 1/2 \times \text{longer diameter (mm)} \times [\text{shorter diameter (mm)}]^2$$

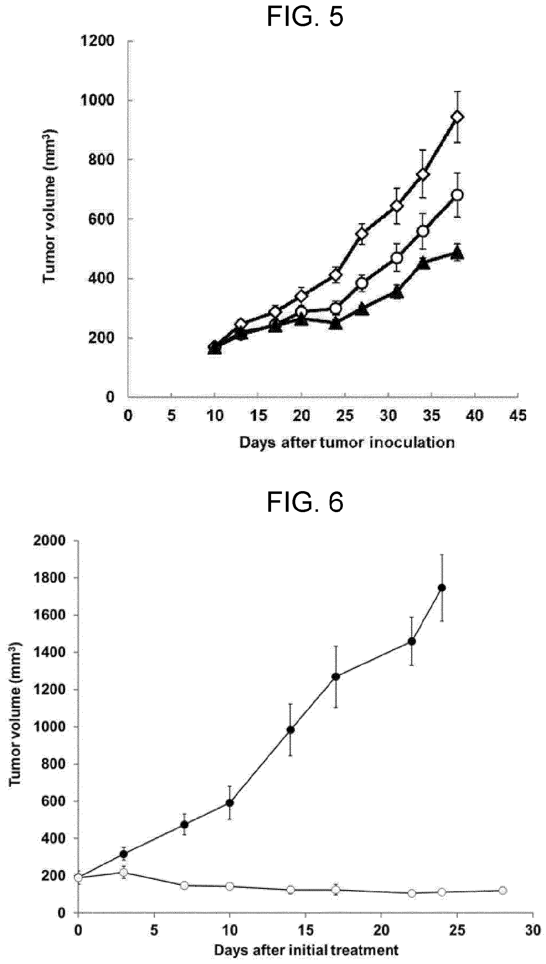

All of the anti-B7-H3 antibody-drug conjugate (1) was diluted with 10 mM acetate buffer (pH 5.5) and 5% sorbitol (ABS buffer) and intravenously administered into the tail vein at a solution volume of 10 mL/kg. The human mesothelioma cell line MSTO-211H cells were purchased from ATCC (American Type Culture Collection). Female nude mice were subcutaneously inoculated with $2.5 \times 10^6$ cells suspended in Matrigel on the right side of the abdomen on Day 0 and then randomly grouped on Day 10. The anti-B7-H3 antibody-drug conjugate (1) was intravenously administered at a dose of 3 mg/kg or 10 mg/kg to the tail vein on Days 10 and 24.
The results are shown in Figure 5. In the figure, white diamonds represent the ABS buffer treated group, white circles represent the 3 mg/kg anti-B7-H3 antibody-drug conjugate (1) treated group, and black triangles represent the 10 mg/kg anti-B7-H3 antibody-drug conjugate (1) treated group. The anti-B7-H3 antibody-drug conjugate (1) exerted inhibitory effects on tumor growth in a dosage-dependent manner.

Example 3: Antitumor activity of the anti-B7-H3 antibody-drug conjugate (1) on a patient-derived xenograft (PDX) model

A study using the PDX model was carried out at Champions Oncology, Inc. The PDX model (CTG-0967) was established by subcutaneously implanting female Hsd: Athymic Nude-Foxn1$^{nu}$ mice with tumor fragments derived from a mesothelioma patient, which were maintained in host mice. A longer diameter and a shorter diameter of the tumor were measured twice a week using a digital caliper, and a tumor volume ($mm^3$) was calculated by the following formula.

$$\text{Tumor volume } (mm^3) = 0.52 \times \text{longer diameter (mm)} \times [\text{shorter diameter (mm)}]^2$$

Group assignment was carried out when the tumor volume reached approximately 150 to 300 $mm^3$ (n = 6 mice/group, Day 0). The ABS buffer was used as a vehicle control and a diluent of the anti-B7-H3 antibody-drug conjugate (1). The ABS buffer or anti-B7-H3 antibody-drug conjugate (1) (10 mg/kg) was intravenously administered to the tail vein

of the mice on Days 0 and 14.

The data in Figure 6 represent the mean tumor volume ± standard error of the mean in each group. In the mesothelioma PDX models (CTG-0967), the anti-B7-H3 antibody-drug conjugate (1) (white circles) demonstrated a strong antitumor effect as compared with the ABS buffer (black circles).

These results suggested that the anti-B7-H3 antibody-drug conjugate (1) shows antitumor effects in mesotheliomas.

Free Text of Sequence Listing

SEQ ID NO: 1 - Amino acid sequence of B7-H3 variant 1
SEQ ID NO: 2 - Amino acid sequence of B7-H3 variant 2
SEQ ID NO: 3 - Amino acid sequence of a heavy chain of the anti-B7-H3 antibody (M30-H1 type)
SEQ ID NO: 4 - Amino acid sequence of a light chain of the anti-B7-H3 antibody (M30-L4 type)
SEQ ID NO: 5 - Amino acid sequence of a heavy chain of the anti-B7-H3 antibody (M30-H2 type)
SEQ ID NO: 6 - Amino acid sequence of a heavy chain of the anti-B7-H3 antibody (M30-H3 type)
SEQ ID NO: 7 - Amino acid sequence of a heavy chain of the anti-B7-H3 antibody (M30-H4 type)
SEQ ID NO: 8 - Amino acid sequence of a light chain of the anti-B7-H3 antibody (M30-L1 type)
SEQ ID NO: 9 - Amino acid sequence of a light chain of the anti-B7-H3 antibody (M30-L2 type)
SEQ ID NO: 10 - Amino acid sequence of a light chain of the anti-B7-H3 antibody (M30-L3 type)
SEQ ID NO: 11 - Amino acid sequence of a light chain of the anti-B7-H3 antibody (M30-L5 type)
SEQ ID NO: 12 - Amino acid sequence of a light chain of the anti-B7-H3 antibody (M30-L6 type)
SEQ ID NO: 13 - Amino acid sequence of a light chain of the anti-B7-H3 antibody (M30-L7 type)

## Claims

1. A therapeutic agent for mesothelioma comprising, as an active component, an anti-B7-H3 antibody-drug conjugate in which a drug-linker represented by the following formula:

[Formula 1]

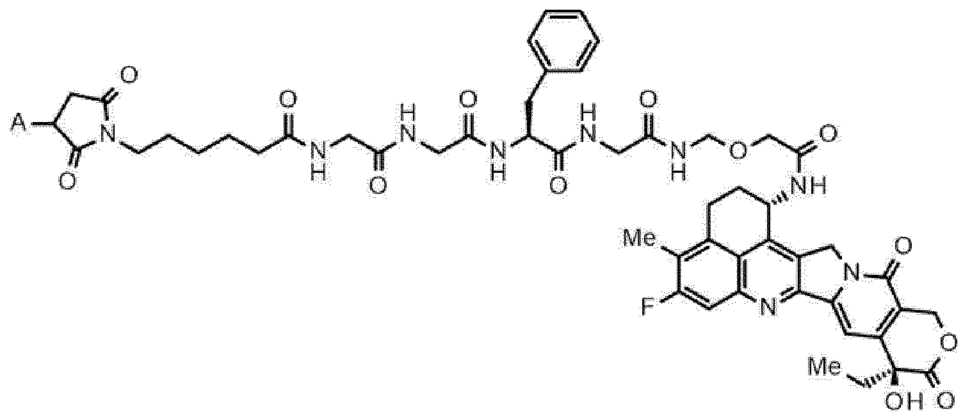

wherein A represents a connecting position to an anti-B7-H3 antibody;
is conjugated to the anti-B7-H3 antibody via a thioether bond.

2. The therapeutic agent according to claim 1, wherein the mesothelioma is at least one selected from the group consisting of pleural mesothelioma, peritoneal mesothelioma, pericardial mesothelioma, and tunica vaginalis testis mesothelioma.

3. The therapeutic agent according to claim 1, wherein the mesothelioma is pleural mesothelioma and/or peritoneal mesothelioma.

4. The therapeutic agent according to claim 1, wherein the mesothelioma is pleural mesothelioma.

5. The therapeutic agent according to any one of claims 1 to 4, wherein the anti-B7-H3 antibody is an antibody comprising a heavy chain comprising CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3, and a light chain comprising CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

6. The therapeutic agent according to any one of claims 1 to 4, wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, or an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

7. The therapeutic agent according to claim 6, wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

8. The therapeutic agent according to any one of claims 1 to 4, wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino

acid sequence consisting of amino acid residues 21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

**9.** The therapeutic agent according to claim 8, wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

**10.** The therapeutic agent according to any one of claims 1 to 9, wherein a lysine residue at the carboxyl terminus of the heavy chain of the anti-B7-H3 antibody is deleted.

**11.** The therapeutic agent according to any one of claims 1 to 10, wherein the average number of units of the drug-linker conjugated per antibody molecule in the anti-B7-H3 antibody-drug conjugate is in the range of from 3.5 to 4.5.

**12.** A therapeutic agent for mesothelioma comprising, as an active component, an anti-B7-H3 antibody-drug conjugate represented by the following formula:

[Formula 2]

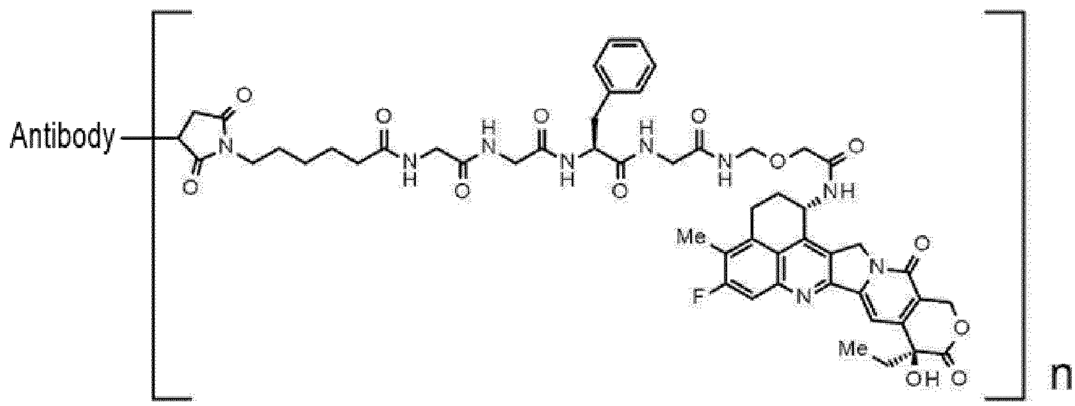

wherein a drug-linker is conjugated to an antibody via a thioether bond, the antibody represents an anti-B7-H3 antibody, and n represents the average number of units of the drug-linker conjugated per antibody molecule.

**13.** The therapeutic agent according to claim 12, wherein the mesothelioma is at least one selected from the group consisting of pleural mesothelioma, peritoneal mesothelioma, pericardial mesothelioma, and tunica vaginalis testis mesothelioma.

**14.** The therapeutic agent according to claim 12, wherein the mesothelioma is pleural mesothelioma and/or peritoneal mesothelioma.

**15.** The therapeutic agent according to claim 12, wherein the mesothelioma is pleural mesothelioma.

**16.** The therapeutic agent according to any one of claims 12 to 15, wherein the anti-B7-H3 antibody is an antibody comprising a heavy chain comprising CDRH1 consisting of an amino acid sequence consisting of amino acid residues 50 to 54 of SEQ ID NO: 3, CDRH2 consisting of an amino acid sequence consisting of amino acid residues 69 to 85 of SEQ ID NO: 3, and CDRH3 consisting of an amino acid sequence consisting of amino acid residues 118 to 130 of SEQ ID NO: 3, and a light chain comprising CDRL1 consisting of an amino acid sequence consisting of amino acid residues 44 to 53 of SEQ ID NO: 4, CDRL2 consisting of an amino acid sequence consisting of amino acid residues 69 to 75 of SEQ ID NO: 4, and CDRL3 consisting of an amino acid sequence consisting of amino acid residues 108 to 116 of SEQ ID NO: 4.

**17.** The therapeutic agent according to any one of claims 12 to 15, wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 10, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 11, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 12, an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 13, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 8, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 9, an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 10, or an antibody consisting of a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 4.

**18.** The therapeutic agent according to claim 17, wherein the anti-B7-H3 antibody is an antibody consisting of a heavy chain comprising a heavy chain variable region consisting of an amino acid sequence consisting of amino acid residues 20 to 141 of SEQ ID NO: 3 and a light chain comprising a light chain variable region consisting of an amino acid sequence consisting of amino acid residues 21 to 128 of SEQ ID NO: 4.

**19.** The therapeutic agent according to any one of claims 12 to 15, wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 3 and a light chain consisting of an amino

acid sequence consisting of amino acid residues 21 to 233 of SEQ ID NO: 10, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 11, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 12, an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 13, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 9, an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 10, or an antibody consisting of a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 4.

**20.** The therapeutic agent according to claim 19, wherein the anti-B7-H3 antibody is an antibody consisting of a heavy chain consisting of an amino acid sequence consisting of amino acid residues 20 to 471 of SEQ ID NO: 3 and a light chain consisting of an amino acid sequence consisting of amino acid residues 21 to 233 of SEQ ID NO: 4.

**21.** The therapeutic agent according to any one of claims 12 to 20, wherein a lysine residue at the carboxyl terminus of the heavy chain of the anti-B7-H3 antibody is deleted.

**22.** The therapeutic agent according to any one of claims 12 to 21, wherein the average number of units of the drug-linker conjugated per antibody molecule in the anti-B7-H3 antibody-drug conjugate is in the range of from 3.5 to 4.5.

# FIG. 1

Amino acid sequence of the B7-H3 variant 1 (SEQ ID NO: 1)

M L R R R G S P G M G V H V G A A L G A L W F C L T G A L E V Q V P E D P V V A L V G T D
A T L C C S F S P E P G F S L A Q L N L I W Q L T D T K Q L V H S F A E G Q D Q G S A Y A
N R T A L F P D L L A Q G N A S L R L Q R V R V A D E G S F T C F V S I R D F G S A A V S
L Q V A A P Y S K P S M T L E P N K D L R P G D T V T I T C S S Y Q G Y P E A E V F W Q D
G Q G V P L T G N V T T S Q M A N E Q G L F D V H S I L R V V L G A N G T Y S C L V R N P
V L Q Q D A H S S V T I T P Q R S P T G A V E V Q V P E D P V V A L V G T D A T L R C S F
S P E P G F S L A Q L N L I W Q L T D T K Q L V H S F T E G R D Q G S A Y A N R T A L F P
D L L A Q G N A S L R L Q R V R V A D E G S F T C F V S I R D F G S A A V S L Q V A A P Y
S K P S M T L E P N K D L R P G D T V T I T C S S Y R G Y P E A E V F W Q D G Q G V P L T
G N V T T S Q M A N E Q G L F D V H S V L R V V L G A N G T Y S C L V R N P V L Q Q D A H
G S V T I T G Q P M T F P P E A L W V T V G L S V C L I A L L V A L A F V C W R K I K Q S
C E E E N A G A E D Q D G E G E G S K T A L Q P L K H S D S K E D D G Q E I A

# FIG. 2

Amino acid sequence of the B7-H3 variant 2 (SEQ ID NO: 2)

M L R R R G S P G M G V H V G A A L G A L W F C L T G A L E V Q V P E D P V V A L V G T D
A T L C C S F S P E P G F S L A Q L N L I W Q L T D T K Q L V H S F A E G Q D Q G S A Y A
N R T A L F P D L L A Q G N A S L R L Q R V R V A D E G S F T C F V S I R D F G S A A V S
L Q V A A P Y S K P S M T L E P N K D L R P G D T V T I T C S S Y R G Y P E A E V F W Q D
G Q G V P L T G N V T T S Q M A N E Q G L F D V H S V L R V V L G A N G T Y S C L V R N P
V L Q Q D A H G S V T I T G Q P M T F P P E A L W V T V G L S V C L I A L L V A L A F V C
W R K I K Q S C E E E N A G A E D Q D G E G E G S K T A L Q P L K H S D S K E D D G Q E I
A

# FIG. 3

SEQ ID NO: 3 - Amino acid sequence of a heavy chain of the anti-B7-H3 antibody (M30-H1 type)

M K H L W F F L L L V A A P R W V L S Q V Q L V Q S G A E V K K P G S S V K
V S C K A S G Y T F T N Y V M H W V R Q A P G Q G L E W M G Y I N P Y N D D
V K Y N E K F K G R V T I T A D E S T S T A Y M E L S S L R S E D T A V Y Y
C A R W G Y Y G S P L Y Y F D Y W G Q G T L V T V S S A S T K G P S V F P L
A P S S K S T S G G T A A L G C L V K D Y F P E P V T V S W N S G A L T S G
V H T F P A V L Q S S G L Y S L S S V V T V P S S S L G T Q T Y I C N V N H
K P S N T K V D K R V E P K S C D K T H T C P P C P A P E L L G G P S V F L
F P P K P K D T L M I S R T P E V T C V V V D V S H E D P E V K F N W Y V D
G V E V H N A K T K P R E E Q Y N S T Y R V V S V L T V L H Q D W L N G K E
Y K C K V S N K A L P A P I E K T I S K A K G Q P R E P Q V Y T L P P S R E
E M T K N Q V S L T C L V K G F Y P S D I A V E W E S N G Q P E N N Y K T T
P P V L D S D G S F F L Y S K L T V D K S R W Q Q G N V F S C S V M H E A L
H N H Y T Q K S L S L S P G K

Signal sequence (1-19), Variable region (20-141), Constant region (142-471)

# FIG. 4

SEQ ID NO: 4 - Amino acid sequence of a light chain of the anti-B7-H3 antibody (M30-L4 type)

M V L Q T Q V F I S L L L W I S G A Y G E I V L T Q S P A T L S L S P G E R
A T L S C R A S S R L I Y M H W Y Q Q K P G Q A P R P L I Y A T S N L A S G
I P A R F S G S G S G T D F T L T I S S L E P E D F A V Y Y C Q Q W N S N P
P T F G Q G T K V E I K R T V A A P S V F I F P P S D E Q L K S G T A S V V
C L L N N F Y P R E A K V Q W K V D N A L Q S G N S Q E S V T E Q D S K D S
T Y S L S S T L T L S K A D Y E K H K V Y A C E V T H Q G L S S P V T K S F
N R G E C

Signal sequence (1-20), Variable region (21-128), Constant region (129-233)

FIG. 5

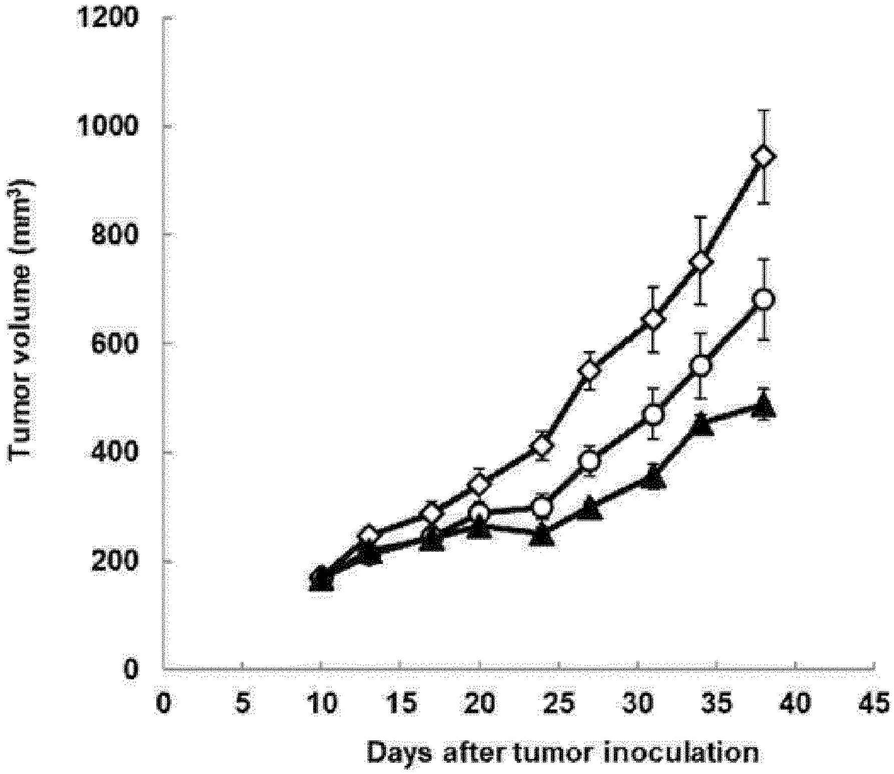
1200
1000
800
600
400
200
0
Tumor volume (mm³)
0
5
10
15
20
25
30
35
40
45
Days after tumor inoculation

FIG. 6

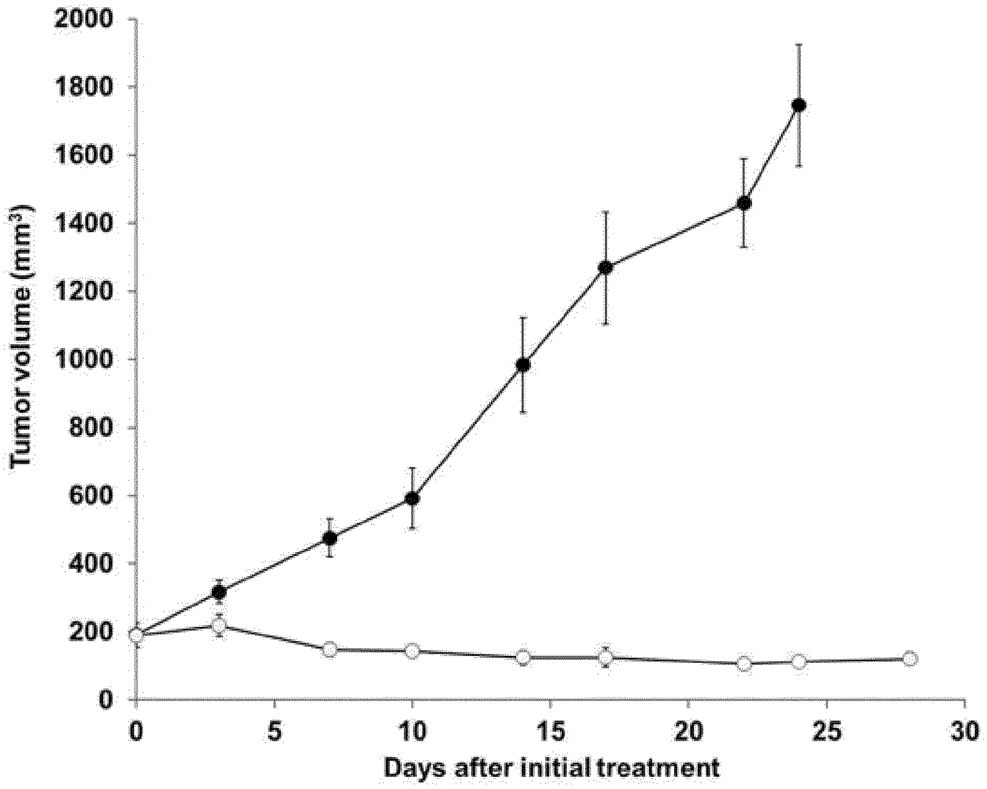
2000
1800
1600
1400
1200
1000
800
600
400
200
0
Tumor volume (mm³)
0
5
10
15
20
25
30
Days after initial treatment

# FIG. 7

SEQ ID NO: 5 - Amino acid sequence of a heavy chain of the anti-B7-H3 antibody (M30-H2 type)

M K H L W F F L L L V A A P R W V L S Q V Q L V Q S G A E V K K P G S S V K V S C K A S G
Y T F T N Y V M H W V R Q A P G Q G L E W I G Y I N P Y N D D V K Y N E K F K G R V T I T
A D E S T S T A Y M E L S S L R S E D T A V Y Y C A R W G Y Y G S P L Y Y F D Y W G Q G T
L V T V S S A S T K G P S V F P L A P S S K S T S G G T A A L G C L V K D Y F P E P V T V
S W N S G A L T S G V H T F P A V L Q S S G L Y S L S S V V T V P S S S L G T Q T Y I C N
V N H K P S N T K V D K R V E P K S C D K T H T C P P C P A P E L L G G P S V F L F P P K
P K D T L M I S R T P E V T C V V V D V S H E D P E V K F N W Y V D G V E V H N A K T K P
R E E Q Y N S T Y R V V S V L T V L H Q D W L N G K E Y K C K V S N K A L P A P I E K T I
S K A K G Q P R E P Q V Y T L P P S R E E M T K N Q V S L T C L V K G F Y P S D I A V E W
E S N G Q P E N N Y K T T P P V L D S D G S F F L Y S K L T V D K S R W Q Q G N V F S C S
V M H E A L H N H Y T Q K S L S L S P G K

Signal sequence (1-19), Variable region (20-141), Constant region (142-471)

# FIG. 8

SEQ ID NO: 6 - Amino acid sequence of a heavy chain of the anti-B7-H3 antibody (M30-H3 type)

M K H L W F F L L L V A A P R W V L S E V Q L V Q S G A E V K K P G S S V K V S C K A S G
Y T F T N Y V M H W V K Q A P G Q G L E W I G Y I N P Y N D D V K Y N E K F K G K A T I T
A D E S T S T A Y M E L S S L R S E D T A V Y Y C A R W G Y Y G S P L Y Y F D Y W G Q G T
L V T V S S A S T K G P S V F P L A P S S K S T S G G T A A L G C L V K D Y F P E P V T V
S W N S G A L T S G V H T F P A V L Q S S G L Y S L S S V V T V P S S S L G T Q T Y I C N
V N H K P S N T K V D K R V E P K S C D K T H T C P P C P A P E L L G G P S V F L F P P K
P K D T L M I S R T P E V T C V V V D V S H E D P E V K F N W Y V D G V E V H N A K T K P
R E E Q Y N S T Y R V V S V L T V L H Q D W L N G K E Y K C K V S N K A L P A P I E K T I
S K A K G Q P R E P Q V Y T L P P S R E E M T K N Q V S L T C L V K G F Y P S D I A V E W
E S N G Q P E N N Y K T T P P V L D S D G S F F L Y S K L T V D K S R W Q Q G N V F S C S
V M H E A L H N H Y T Q K S L S L S P G K

Signal sequence (1-19), Variable region (20-141), Constant region (142-471)

# FIG. 9

SEQ ID NO: 7 - Amino acid sequence of a heavy chain of the anti-B7-H3 antibody (M30-H4 type)

MKHLWFFLLLVAAPRWVLSEVQLVQSGAEVKKPGSSVKVSCKASG
YTFTNYVMHWVKQAPGQGLEWIGYINPYNDDVKYNEKFKGKATQT
SDKSTSTAYMELSSLRSEDTAVYYCARWGYYGSPLYYFDYWGQGT
LVTVSSASTKGPSVFPLAPSSKSTSGGTAALGCLVKDYFPEPVTV
SWNSGALTSGVHTFPAVLQSSGLYSLSSVVTVPSSSLGTQTYICN
VNHKPSNTKVDKRVEPKSCDKTHTCPPCPAPELLGGPSVFLFPPK
PKDTLMISRTPEVTCVVVDVSHEDPEVKFNWYVDGVEVHNAKTKP
REEQYNSTYRVVSVLTVLHQDWLNGKEYKCKVSNKALPAPIEKTI
SKAKGQPREPQVYTLPPSREEMTKNQVSLTCLVKGFYPSDIAVEW
ESNGQPENNYKTTPPVLDSDGSFFLYSKLTVDKSRWQQGNVFSCS
VMHEALHNHYTQKSLSLSPGK

Signal sequence (1-19), Variable region (20-141), Constant region (142-471)

# FIG. 10

SEQ ID NO: 8 - Amino acid sequence of a light chain of the anti-B7-H3 antibody (M30-L1 type)

MVLQTQVFISLLLWISGAYGEIVLTQSPATLSLSPGERATLSCRA
SSRLIYMHWYQQKPGQAPRLLIYATSNLASGIPARFSGSGSGTDF
TLTISRLEPEDFAVYYCQQWNSNPPTFGQGTKVEIKRTVAAPSVF
IFPPSDEQLKSGTASVVCLLNNFYPREAKVQWKVDNALQSGNSQE
SVTEQDSKDSTYSLSSTLTLSKADYEKHKVYACEVTHQGLSSPVT
KSFNRGEC

Signal sequence (1-20), Variable region (21-128), Constant region (129-233)

# FIG. 11

SEQ ID NO: 9 - Amino acid sequence of a light chain of the anti-B7-H3 antibody (M30-L2 type)

MVLQTQVFISLLLWISGAYGEIVLTQSPATLSLSPGERATLSCRA
SSRLIYMHWYQQKPGQAPRLWIYATSNLASGIPARFSGSGSGTDY
TLTISRLEPEDFAVYYCQQWNSNPPTFGQGTKVEIKRTVAAPSVF
IFPPSDEQLKSGTASVVCLLNNFYPREAKVQWKVDNALQSGNSQE
SVTEQDSKDSTYSLSSTLTLSKADYEKHKVYACEVTHQGLSSPVT
KSFNRGEC

Signal sequence (1-20), Variable region (21-128), Constant region (129-233)

# FIG. 12

SEQ ID NO: 10 - Amino acid sequence of a light chain of the anti-B7-H3 antibody (M30-L3 type)

MVLQTQVFISLLLWISGAYGQIVLSQSPATLSLSPGERATLTCRA
SSRLIYMHWYQQKPGSAPKLWIYATSNLASGIPARFSGSGSGTSY
TLTISRLEPEDFAVYYCQQWNSNPPTFGQGTKVEIKRTVAAPSVF
IFPPSDEQLKSGTASVVCLLNNFYPREAKVQWKVDNALQSGNSQE
SVTEQDSKDSTYSLSSTLTLSKADYEKHKVYACEVTHQGLSSPVT
KSFNRGEC

Signal sequence (1-20), Variable region (21-128), Constant region (129-233)

# FIG. 13

SEQ ID NO: 11 - Amino acid sequence of a light chain of the anti-B7-H3 antibody (M30-L5 type)

MVLQTQVFISLLLWISGAYGQIVLSQSPATLSLSPGERATLTCRA
SSRLIYMHWYQQKPGSAPKPWIYATSNLASGIPARFSGSGSGTSY
TLTISRLEPEDFAVYYCQQWNSNPPTFGQGTKVEIKRTVAAPSVF
IFPPSDEQLKSGTASVVCLLNNFYPREAKVQWKVDNALQSGNSQE
SVTEQDSKDSTYSLSSTLTLSKADYEKHKVYACEVTHQGLSSPVT
KSFNRGEC

Signal sequence (1-20), Variable region (21-128), Constant region (129-233)

# FIG. 14

SEQ ID NO: 12 - Amino acid sequence of a light chain of the anti-B7-H3 antibody (M30-L6 type)

MVLQTQVFISLLLWISGAYGEIVLTQSPATLSLSPGERATLSCRA
SSRLIYMHWYQQKPGQAPRPLIYATSNLASGIPARFSGSGSGTDF
TLTISRLEPEDFAVYYCQQWNSNPPTFGQGTKVEIKRTVAAPSVF
IFPPSDEQLKSGTASVVCLLNNFYPREAKVQWKVDNALQSGNSQE
SVTEQDSKDSTYSLSSTLTLSKADYEKHKVYACEVTHQGLSSPVT
KSFNRGEC

Signal sequence (1-20), Variable region (21-128), Constant region (129-233)

# FIG. 15

SEQ ID NO: 13 - Amino acid sequence of a light chain of the anti-B7-H3 antibody (M30-L7 type)

MVLQTQVFISLLLWISGAYGEIVLTQSPATLSLSPGERATLSCRA
SSRLIYMHWYQQKPGQAPRPLIYATSNLASGIPARFSGSGSGTDY
TLTISRLEPEDFAVYYCQQWNSNPPTFGQGTKVEIKRTVAAPSVF
IFPPSDEQLKSGTASVVCLLNNFYPREAKVQWKVDNALQSGNSQE
SVTEQDSKDSTYSLSSTLTLSKADYEKHKVYACEVTHQGLSSPVT
KSFNRGEC

Signal sequence (1-20), Variable region (21-128), Constant region (129-233)

## INTERNATIONAL SEARCH REPORT

International application No.
**PCT/JP2021/041496**

**A. CLASSIFICATION OF SUBJECT MATTER**

***A61K 47/68***(2017.01)i; ***A61K 31/4745***(2006.01)i; ***A61K 39/395***(2006.01)i; ***A61P 35/00***(2006.01)i
FI: A61K47/68; A61P35/00 ZNA; A61K31/4745; A61K39/395 T; A61K39/395 W

According to International Patent Classification (IPC) or to both national classification and IPC

**B. FIELDS SEARCHED**

Minimum documentation searched (classification system followed by classification symbols)

A61K47/68; A61K31/4745; A61K39/395; A61P35/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); GenBank/EMBL/DDBJ/GeneSeq; UniProt/GeneSeq; SwissProt/GeneSeq

**C. DOCUMENTS CONSIDERED TO BE RELEVANT**

| Category* | Citation of document, with indication, where appropriate, of the relevant passages | Relevant to claim No. |
|---|---|---|
| Y | WO 2014/057687 A1 (DAIICHI SANKYO CO., LTD.) 17 April 2014 (2014-04-17) claims 15, 21, 24, 29, 31, paragraphs [0003]-[0005], [0072]-[0076], [0086] | 1-22 |
| Y | WO 2017/002776 A1 (DAIICHI SANKYO CO., LTD.) 05 January 2017 (2017-01-05) claims 1, 16-17, 19-22, paragraphs [0071]-[0073], [0088] | 1-22 |
| Y | CALABRO, L. et al., Expression and Regulation of B7-H3 Immunoregulatory Receptor, in Human Mesothelial and Mesothelioma Cells: Immunotherapeutic Implications, Journal of Cellular Physiology, 28 December 2010, vol. 226, pp. 2595-2600 abstract, p. 2598, left column, paragraph 2 to right column, paragraph 1, p. 2600, left column, paragraph 2, fig. 4, 5 | 1-22 |
| Y | WO 2017/141604 A1 (KANAGAWA PREFECTURAL HOSPITAL ORGANIZATION) 24 August 2017 (2017-08-24) paragraph [0013] | 2-4, 13-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 December 2021** | **14 December 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2021/041496**

**Box No. I** **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/041496**

| Patent document cited in search report | Publication date (day/month/year) | Patent family member(s) | Publication date (day/month/year) |
|---|---|---|---|
| WO 2014/057687 A1 | 17 April 2014 | US 2015/0297748 A1<br>claims 15, 21, 24, 29, 31, paragraphs [0003], [0004], [0223]-[0232], [0257]<br>EP 2907824 A1<br>KR 10-2015-0067149 A<br>CN 104755494 A | |
| WO 2017/002776 A1 | 05 January 2017 | US 2018/0147292 A1<br>claims 1, 16-17, 19-22, paragraphs [0167]-[0169], [0184]<br>EP 3315512 A1<br>KR 10-2018-0021723 A<br>CN 107922477 A | |
| WO 2017/141604 A1 | 24 August 2017 | US 2019/0071517 A1<br>paragraph [0030]<br>EP 3418304 A1<br>CN 108699159 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014057687 A
- WO 2017002776 A
- WO 9007861 A
- US 5821337 A
- WO 9954342 A
- WO 0061739 A
- WO 0231140 A
- WO 200713385 A
- WO 2013120066 A
- WO 2015098099 A
- WO 2015115091 A
- WO 2015155998 A
- WO 2019044947 A


### Non-patent literature cited in the description


- **TIMOTHY A YAP. et al.** *Nat Rev Cancer.,* 2017, vol. 17 (8), 475-488
- **CARBONE M. et al.** *Ca Cancer J Clin,* 2019, vol. 69, 402-429
- **PICARDA E. et al.** *Clin Cancer Res.,* 2016, vol. 22 (14), 3425-31
- **LING V. et al.** *Genomics,* 2003, vol. 82 (3), 365-77
- **DUCRY L. et al.** *Bioconjugate Chem.,* 2010, vol. 21, 5-13
- **ALLEY S. C. et al.** *Current Opinion in Chemical Biology,* 2010, vol. 14, 529-537
- **DAMLE N. K.** *Expert Opin. Biol. Ther.,* 2004, vol. 4, 1445-1452
- **SENTER P. D. et al.** *Nature Biotechnology,* 2012, vol. 30, 631-637
- **HOWARD A. et al.** *J Clin Oncol,* vol. 29, 398-405
- **OGITANI Y. et al.** *Clinical Cancer Research,* 29 March 2016, vol. 22 (20), 5097-5108
- **OGITANI Y. et al.** *Cancer Science,* 2016, vol. 107, 1039-1046
- *Cell Death and Differentiation,* 2008, vol. 15, 751-761
- *Molecular Biology of the Cell,* December 2004, vol. 15, 5268-5282
- *Bio Techniques,* January 2000, vol. 28, 162-165
- **KOHLER ; MILSTEIN.** *Nature,* 1975, vol. 256, 495-497
- Monoclonal Antibodies. Plenum Press, 1980, 365-367
- *Proc. Natl. Acad. Sci. USA,* 1984, vol. 81, 6851-6855
- *Nature,* 1986, vol. 321, 522-525
- **TOMIZUKA, K. et al.** *Nature Genetics,* 1997, vol. 16, 133-143
- **KUROIWA, Y.** *Nucl. Acids Res.,* 1998, vol. 26, 3447-3448
- **YOSHIDA, H.** Animal Cell Technology: Basic and Applied Aspects. Kluwer Academic Publishers, 1999, vol. 10, 69-73
- **TOMIZUKA, K.** *Proc. Natl. Acad. Sci. USA,* 2000, vol. 97, 722-727
- **WORMSTONE, I. M.** *Investigative Ophthalmology & Visual Science.,* 2002, vol. 43 (7), 2301-2308
- **CARMEN, S.** *Briefings in Functional Genomics and Proteomics,* 2002, vol. 1 (2), 189-203
- **SIRIWARDENA, D.** *Ophthalmology,* 2002, vol. 109 (3), 427-431
- *Journal of Chromatography A,* 1995, vol. 705, 129-134
- *Analytical Biochemistry,* 2007, vol. 360, 75-83
- **ALTSCHUL, STEPHEN F. ; THOMAS L. MADDEN ; ALEJANDRO A. SCHAFFER ; JINGHUI ZHANG ; ZHENG ZHANG ; WEBB MILLER ; DAVID J. LIPMAN.** Gapped BLAST and PSI-BLAST: a new generation of protein database search programs. *Nucleic Acids Res.,* 1997, vol. 25, 3389-3402
- **HERMANSON, G. T.** Bioconjugate Techniques. Academic Press, 1996, 56-136, 456-493